# FASCICULE DE BREVET EUROPEEN

(11) **EP 3 784 662 B1**
(45) Date de publication et mention de la délivrance du brevet: **09.11.2022**
(21) Numéro de dépôt: 19733845.2
(22) Date de dépôt: 26.04.2019
(51) Int. Cl.: C07D 401/12, C07D 403/12, A61K 31/473, A61K 31/4725, A61K 31/517, A61K 31/4709, A61K 31/4706, A61P 35/00

(54) **COMPOSES A ACTIVITE INHIBITRICE DE LA POLYMERISATION DE LA TUBULINE ET AUX PROPRIETES IMMUNOMODULATRICES**
VERBINDUNGEN MIT EINER TUBULIN-POLYMERISATIONSHEMMENDEN AKTIVITÄT UND IMMUNMODULATORISCHEN EIGENSCHAFTEN
COMPOUNDS HAVING A TUBULIN-POLYMERISATION-INHIBITING ACTIVITY AND IMMUNOMODULATORY PROPERTIES

(30) Priorité: 27.04.2018 FR 1853709
(43) Date de publication de la demande: 03.03.2021
(73) Titulaire: Université Paris-Saclay, 91190 Gif-sur-Yvette (FR); Centre National de la Recherche Scientifique (CNRS), 75016 Paris (FR); Institut Gustave-Roussy, 94800 Villejuif (FR); INSTITUT NATIONAL DE LA SANTE ET DE LA RECHERCHE MEDICALE (INSERM), 75013 Paris (FR)
(72) Inventeur: ALAMI, Mouad, 77600 Bussy Saint Georges (FR); PROVOT, Olivier, 78500 Sartrouville (FR); HAMZE, Abdallah, 91120 Palaiseau (FR); KHELIFI, Ilhem, 93340 Le Raincy (FR); NARET, Timothée, 39700 Orchamps (FR); APCHER, Sébastien, 95130 Franconville (FR); DARRIGRAND, Romain, 11100 Narbonne (FR)
(74) Mandataire: Regimbeau
(86) Numéro de dépôt international: PCT/FR2019/050982
(87) Numéro de publication internationale: WO 2019/207257

(56) Documents cités:
- MAHAL K. ET AL.: "Effects of the Tumor-Vasculature-Disrupting Agent Verubulin and Two Heteroaryl Analogues on Cancer Cells, Endothelial Cells, and Blood Vessels", CHEMMEDCHEM, vol. 9, no. 4, 1 janvier 2014 (2014-01-01) , pages 847-854, XP002784057,

## Description

### DOMAINE DE L'INVENTION

La présente invention concerne des composés agissant comme agents antivasculaires par inhibition de la polymérisation de la tubuline et/ou ayant des propriétés immunomodulatrices, leur procédé de préparation ainsi que leur utilisation dans le traitement du cancer.

### ETAT DE LA TECHNIQUE

Le cancer est la première cause de mortalité chez l'homme âgé de moins de 65 ans, la deuxième chez la femme. Plus de 2 millions de nouveaux cas sont diagnostiqués annuellement en Europe. Le nombre de décès par cancers en France est estimé à 150 000 en 2015. L'impact du cancer sur la société demeure ainsi considérable.

Les grands types de traitements contre le cancer sont : la chirurgie, la radiothérapie, la chimiothérapie dite conventionnelle (impliquant des agents cytotoxiques), les thérapies ciblées (visant spécifiquement certains mécanismes impliqués dans la régulation et la croissance cellulaire), l'hormonothérapie (adaptée dans le cas de cancers sensibles à l'action d'hormones naturellement produites par l'organisme), l'immunothérapie (visant à stimuler le système immunitaire du malade contre les cellules tumorales).

La chimiothérapie conventionnelle impliquant des agents cytotoxiques, seule ou associée à la chirurgie ou la radiothérapie occupe une place majeure. Toutefois, les traitements sont fréquemment accompagnés d'effets indésirables par manque de sélectivité. De plus, la multi-résistance, le principal mécanisme par lequel de nombreux cancers échappent aux traitements, est un facteur important de l'échec de nombreuses chimiothérapies.

Les progrès récents accomplis dans le traitement des cancers sont liés à l'arrivée des thérapies ciblées. Dans ce contexte, normaliser, détruire ou déréguler la vascularisation tumorale sont les objectifs d'une nouvelle stratégie thérapeutique ciblée qui suscite beaucoup d'espoir. Cette stratégie, visant exclusivement les cellules endothéliales génétiquement plus stables que les cellules cancéreuses, diminue le risque de résistance au traitement. La fosbrétabuline, chef de file des antivasculaires, a reçu en 2016 le statut de médicament orphelin aux Etats-Unis et en Europe pour le traitement de tumeurs neuro-endocrines (NETs) et du glioblastome multiforme (GBM).

Malgré tout l'intérêt des thérapies ciblées, les traitements qui visent une cible unique ont montré des résultats limités en raison de la grande diversité biologique des cancers et l'apparition de phénomènes de résistance. La combinaison de plusieurs principes actifs (ou polychimiothérapie) ayant des mécanismes d'action différents et ciblant les altérations les plus critiques de cette maladie semble judicieuse, à la condition que la toxicité de chaque principe actif pris séparément ne soit pas cumulée. Les pistes de développement s'attachent maintenant à l'utilisation de molécules duales qui inhibent ou modulent plusieurs cibles simultanément. Les inhibiteurs des tyrosines kinases (ITK) représentent un bon exemple de ce concept, capable de bloquer la signalisation des récepteurs VEGF, PDGF et d'autres kinases membranaires et/ou cytoplasmiques. Les avantages de cette stratégie sont multiples : (i) une meilleure efficacité en raison du synergisme de l'effet simultané sur plusieurs cibles, (ii) une seule vitesse de métabolisation, simplifiant les paramètres pharmacocinétiques et pharmacodynamiques, (iii) une baisse voire une absence d'interaction entre les divers principes actifs administrés, etc.

Contrairement aux thérapies ciblées, l'immunothérapie ne vise pas à détruire directement les cellules cancéreuses. Elle vise le système immunitaire afin de renforcer et stimuler les propres défenses du malade contre les cellules cancéreuses. Cette approche a permis la mise sur le marché d'anticorps monoclonaux dits « immunomodulateurs » tels que l'anti-CTLA-4 (ipilimumab/YERVOY^{®}, mélanome métastatique) ou l'anti-PD-1/PD-L1 (pembrolizumab/KEYTRUDA^{®}, nivolumab/OPDIVA^{®}, cancers du rein, poumon etc.). Les immunomodulateurs peuvent être également de petites molécules chimiques agissant de façon ciblée contre un acteur de la réponse immunitaire. A ce jour, seule la thalidomide et ses dérivés ont été décrits comme des molécules immunomodulatrices (myélome multiple). Si ces principes actifs sont utilisés en clinique, il n'en reste pas moins qu'ils sont accompagnés de plusieurs inconvénients dont principalement un effet tératogène, et un risque plus élevé de cancers secondaires (leucémie aiguë myéloblastique et syndromes myélodysplasiques) limitant considérablement leurs utilisations. Par conséquent, la recherche de petites molécules immunomodulatrices est un domaine qui suscite beaucoup d'espoir.

Une stratégie immuno-thérapeutique contre le cancer pourrait consister à cibler la stimulation des lymphocytes T CD8 cytotoxiques. Les lymphocytes T CD8 reconnaissent certains antigènes, petits peptides endogènes de huit à dix acides aminés apprêtés dans une molécule du complexe majeur d'histocompatibilité de classe I (CMH I). Des altérations dans les composants de la machinerie de présentation par le CMH-I ont été mises en évidence dans de nombreux types de cancers, conduisant à une diminution ou une perte de la présentation antigénique et favorisant ainsi l'échappement tumoral. Augmenter la présentation antigénique au sein des tumeurs au moyen de petites molécules permettrait d'améliorer la visibilité des cellules tumorales face aux lymphocytes T CD8. Ces petites molécules auraient alors une activité immunomodulatrice en rendant les cellules tumorales plus visibles vis-à-vis des lymphocytes T CD8.

La combinaison des deux approches thérapeutiques, l'une ciblant la vascularisation tumorale et l'autre le système immunitaire, constitue le rationnel de cette demande de brevet avec l'identification d'une nouvelle classe de composés antitumoraux « multi-cible » agissant à la fois comme antivasculaire et comme immunomodulateur.

Mahal et al. (ChemMedChem 2014, 9, 847-854) décrit des dérivés de quinazoline aux propriétés antiprolifératives, dont le composé 10 ci-dessous :

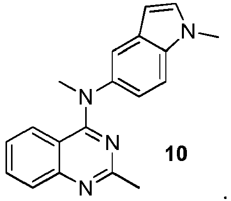

La combrétastatine A-4 (CA-4), molécule d'origine naturelle, est reconnue en tant qu'agent antivasculaire en raison de son aptitude à interagir avec la tubuline des cellules endothéliales activées par les facteurs de croissance dont le facteur de croissance de l'endothélium vasculaire (VEGF) est l'acteur essentiel. Elle détruit sélectivement les vaisseaux irriguant les tumeurs, induisant ainsi une importante nécrose intra-tumorale. La fosbrétabuline, prodrogue de la combrétastatine A-4, a reçu le statut de médicament orphelin aux États-unis et en Europe pour les cancers de la thyroïde et les leucémies myéloides chroniques. D'autres essais cliniques sont en cours, en combinaison avec d'autres agents comme le bévacizumab contre le cancer de l'ovaire résistant. Bien qu'efficace en thérapie, la combrétastatine A-4 est grevée de plusieurs handicaps dont une instabilité chimique, imputable à l'isomérisation de la double liaison Z conduisant à l'isomère inactif E, imposant une conservation à basse température et à l'abri de la lumière.

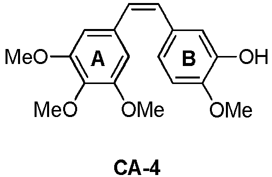

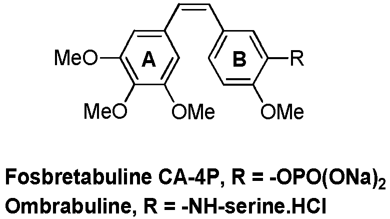

Un moyen de contourner le problème récurrent d'instabilité de la double liaison Z de la CA- 4 a été développé dans la demande WO 2008/122620. L'isocombrétastatine A-4 (*iso*CA-4), et l'isoaminocombrétastatine A-4 (*iso*NH₂CA-4) ont été identifiées comme deux chefs de file dont le profil biologique (cytotoxicité, inhibition de la polymérisation de la tubuline, induction de l'apoptose, etc.) est rigoureusement identique à celui de la molécule naturelle, sans toutefois présenter le risque d'isomérisation de la double liaison. Ces molécules, sont particulièrement stables et ne se métabolisent pas en présence d'hépatocytes.

Poursuivant leurs travaux d'étude de relation structure-activité, les inventeurs ont découvert de manière surprenante des composés ayant non seulement des propriétés cytotoxique et d'inhibition de polymérisation de la tubuline, mais également des propriétés immunomodulatrices. Par ailleurs, l'activité antiproliférative des composés selon l'invention est observée à de très faibles concentrations variant du picomolaire au nanomolaire.

De même, l'activité immunomodulatrice des composés selon l'invention est observée à de très faibles concentrations nanomolaires.

### RESUME DE L'INVENTION

La présente invention a pour objet des composés de formule (I) suivante : 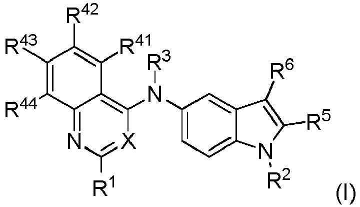 dans laquelle :
- X représente un groupe -CH- ou un atome d'azote,
- R¹ représente un atome d'hydrogène, un atome d'halogène, de préférence un atome de chlore, un groupe (C₁-C₆)alkyle, de préférence un groupe méthyle, un groupe -CN ou un groupe -CF₃,
- R² représente un atome d'hydrogène, un groupe (C₁-C₆)alkyle, un groupe aryle-(Ci-C₆)alkyle, de préférence un benzyle, ou un groupe -COR²¹ avec R²¹ représentant un groupe (C₁-C₆)alkyle ou un groupe aryle,
- R³ représente un atome d'hydrogène, un groupe (C₁-C₆)alkyle, de préférence un groupe méthyle, un groupe aryle-(C₁-C₆)alkyle ou un groupe -COR³¹ avec R³¹ représentant un groupe (C₁-C₆)alkyle ou un groupe aryle ; de préférence un groupe (C₁-C₆)alkyle, de préférence un groupe méthyle, un groupe aryle-(C₁-C₆)alkyle ou un groupe -COR³¹ avec R³¹ représentant un groupe (C₁-C₆)alkyle ou un groupe aryle,
- R⁴¹, R⁴² , R⁴³ et R⁴⁴ représentent indépendamment les uns des autres un atome d'hydrogène ; un atome d'halogène ; -OR⁴⁵ ; -SR⁴⁵ ; -NR⁴⁵R⁴⁶ ; -NO₂; ou un groupe (C₁-C₆)alkyle éventuellement substitué par un ou plusieurs substituants choisis parmi un atome d'halogène, -OR⁴⁵, -SR⁴⁵, -NR⁴⁵R⁴⁶ et -NO₂, avec R⁴⁵ et R⁴⁶ représentant indépendamment l'un de l'autre un atome d'hydrogène, un groupe (C₁-C₆)alkyle, un groupe -CF₃ ou un groupe -COR⁴⁷ avec R⁴⁷ représentant un groupe (C₁-C₆)alkyle ou un groupe aryle,
- R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène ; un atome d'halogène ; -OR⁵⁵ ; -SR⁵⁵ ; -NR⁵⁵R⁵⁶ ; - NO₂; ou un groupe (C₁-C₆)alkyle éventuellement substitué par un ou plusieurs substituants choisis parmi un atome d'halogène,-OR⁵⁵, -SR⁵⁵, -NR⁵⁵R⁵⁶ et -NO₂, avec R⁵⁵ et R⁵⁶ représentant indépendamment l'un de l'autre un atome d'hydrogène, un groupe (C₁-C₆)alkyle, un groupe -CF₃ ou un groupe -COR⁵⁷ avec R⁵⁷ représentant un groupe (C₁-C₆)alkyle ou un groupe aryle.

ou un sel pharmaceutiquement acceptable de ceux-ci,
caractérisé en ce que, lorsque X représente un atome d'azote, R¹ représente un atome de chlore, un groupe -CN ou un groupe -CF₃, ou R⁵ et R⁶ forment ensemble une chaine -CR⁵¹=CR⁵²-CR⁵³=CR⁵⁴-.

La présente invention concerne également un composé (I) tel que décrit ci-dessus ou un sel pharmaceutiquement acceptable de celui-ci pour son utilisation en tant que médicament, notamment destiné à traiter le cancer, en agissant en particulier par inhibition de la polymérisation de la tubuline et/ou par immunomodulation.

La présente invention a également pour objet une composition pharmaceutique comprenant au moins un composé (I) tel que décrit ci-dessus ou un sel pharmaceutiquement acceptable de celui-ci et un excipient pharmaceutiquement acceptable.

La présente invention a également pour objet une composition pharmaceutique comprenant au moins un composé (I) tel que décrit ci-dessus ou un sel pharmaceutiquement acceptable de celui-ci et un excipient pharmaceutiquement acceptable pour son utilisation en tant que médicament, notamment destiné à traiter le cancer, en agissant en particulier par inhibition de la polymérisation de la tubuline et/ou par immunomodulation.

La présente invention concerne également l'utilisation d'un composé (I) tel que décrit ci-dessus ou un sel pharmaceutiquement acceptable de celui-ci pour la préparation d'un médicament destiné au traitement du cancer.

La présente invention concerne également une méthode de traitement du cancer comprenant l'administration à un patient en ayant besoin d'une quantité efficace d'un composé (I) tel que décrit ci-dessus ou un sel pharmaceutiquement acceptable de celui-ci ou d'une composition pharmaceutique telle que définie ci-dessus.

Enfin, la présente invention concerne un procédé de préparation d'un composé (I) tel que décrit ci-dessus ou un sel pharmaceutiquement acceptable de celui-ci comprenant les étapes suivantes
(1) couplage d'un dérivé aminé de formule (II) suivante : 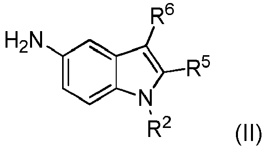 dans laquelle R², R⁵ et R⁶ sont tels que définis ci-dessus,
   avec un composé de formule (III) suivante : 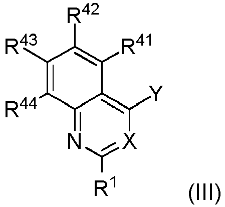 dans laquelle Y représente un atome de chlore, de brome, d'iode, un groupe trifluorométhylsulfonate ou un groupe tosyle et X, R¹, R⁴¹, R⁴², R⁴³ et R⁴⁴ sont tels que définis ci-dessus,
   pour donner un composé de formule (I) tel que décrit ci-dessus avec R³ = H,
(2) éventuellement substitution du groupe R³ = H porté par l'amine du composé de formule (I) obtenu à l'étape (1) qui précède pour donner un composé de formule (I) tel que décrit ci-dessus avec R³ représentant un groupe (C₁-C₆)alkyle, un groupe aryle-(C₁-C₆)alkyle ou un groupe -COR³¹ avec R³¹ tel que défini ci-dessus, et
(3) éventuellement salification du composé de formule (I) obtenu à l'étape (1) ou (2) qui précède pour donner un sel pharmaceutiquement acceptable d'un composé de formule (I) tel que défini ci-dessus.

### DESCRIPTION DES FIGURES

La figure 1 représente le nombre cellules HCT116 en fonction de leur contenu en ADN dans les différentes phases du cycle cellulaire (G0/G1, S, G2/M) après 24 heures de traitement en présence de DMSO (contrôle - véhicule seul) ou en présence du composé 1 à une concentration de 5 nM dans le DMSO.
La figure 2 représente l'induction de l'apoptose dans des cellules HCT116 traitées pendant 24 h avec 1 à diverses concentrations dans le DMSO. L'apoptose est mise en évidence par la mesure de l'activité enzymatique des caspases 3 et 7 et les résultats sont exprimés en % par rapport aux cellules traitées pendant 24 h avec du DMSO à 0,1% (contrôle).

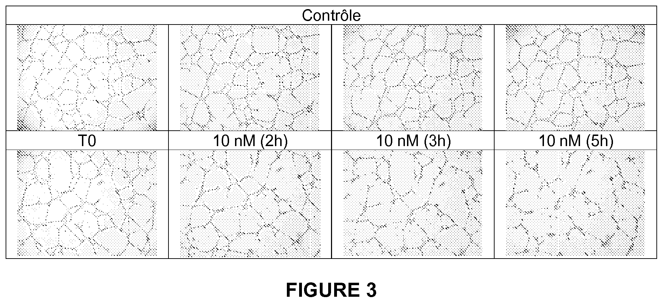

La figure 3 représente l'évolution dans le temps (à t = 0 h, t = 2 h, t = 3 h et t = 5 h) de tubes vasculaires formés par des cellules HUVEC fixées sur Matrigel en présence de DMSO (contrôle) ou en présence du composé 1 à une concentration de 10 nM dans le DMSO.
La figure 4 représente la variation de présentation antigénique vis-à-vis des lymphocytes T CD8 dans la lignée du sarcome MCA205 en fonction de la concentration en composé 18 (figure 4a), **21** (figure 4b) et **10** (figure 4c).
La figure 5 représente la présentation antigénique dans la lignée du mélanome B16F10 en fonction de la concentration en composé **18** (figure 5a), **21** (figure 5b) et **9** (figure 5c).

### DEFINITIONS

Le terme "halogène", tel qu'utilisé dans la description de la présente invention, désigne les atomes de fluor, chlore, brome et iode. De manière avantageuse, il s'agira du fluor, du brome et du chlore et encore plus avantageusement du fluor ou du chlore.

Le terme "(C₁-C₆)alkyle", tel qu'utilisé dans la description de la présente invention, désigne tout groupe hydrocarboné saturé comportant de 1 à 6 atomes de carbone, linéaire ou ramifié, en particulier les groupes méthyle, éthyle, *n*-propyle, isopropyle, *n*-butyle, *iso*-butyle, *sec*-butyle, *tert-*butyle, pentyle et hexyle.

Le terme "aryle", tel qu'utilisé dans la description de la présente invention, désigne un ou plusieurs cycles aromatiques ayant de 5 à 10 atomes de carbone, pouvant être accolés. En particulier, les groupes aryles peuvent être des groupes monocycliques ou bicycliques, comme par exemple le groupe phényle ou naphtyle. Avantageusement, le groupe aryle est un phényle.

Le terme "aryle-(C₁-C₆)alkyle", tel qu'utilisé dans la description de la présente invention, désigne un groupe aryle tel que défini ci-dessus, lié au reste de la molécule par l'intermédiaire d'une chaîne (C₁-C₆)alkyle telle que définie ci-dessus. A titre d'exemple, on peut citer le groupe benzyle ou encore phényléthyle.

L'expression "pharmaceutiquement acceptable", telle qu'utilisée dans la description de la présente invention, désigne ce qui est utile dans la préparation d'une composition pharmaceutique, qui est généralement sûr, non toxique et ni biologiquement ni autrement non souhaitable et qui est acceptable pour une utilisation vétérinaire et/ou pharmaceutique humaine. L'expression "sels pharmaceutiquement acceptables", telle qu'utilisée dans la description de la présente invention, désigne des sels d'un composé qui sont pharmaceutiquement acceptables, comme définis ici, et qui possèdent l'activité pharmacologique souhaitée du composé parent. De tels sels comprennent :
(1) les hydrates et les solvates,
(2) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphosulfonique, l'acide citrique, l'acide éthanesulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide ptoluènesulfonique, l'acide triméthylacétique, l'acide trifluoroacétique et similaires.
   Avantageusement, il s'agit de l'acide chlorhydrique ; ou
(3) les sels formés lorsqu'un proton acide présent dans le composé parent soit est remplacé par un ion métallique, par exemple un ion de métal alcalin, un ion de métal alcalino-terreux ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium. Avantageusement, le proton acide est déplacé par un ion Na+, notamment en utilisant de l'hydroxyde de sodium. Les sels d'addition d'acide sont formés en particulier avec une fonction amine ou avec une pyridine. Les sels d'addition de base sont formés en particulier avec une fonction acide carboxylique (-COOH), phosphate (-OP(O)(OH)₂) ou encore sulfate (-OSO₃H).

Le terme « stéréoisomères », tel qu'utilisé dans la description de la présente invention, désigne des diastéréoisomères ou des énantiomères. Il s'agit donc d'isomères de configuration. Les stéréoisomères qui ne sont pas des images dans un miroir l'un de l'autre sont ainsi désignés par « diastéréoisomères », et les stéréoisomères qui sont des images l'un de l'autre dans un miroir mais non superposables sont désignés par « énantiomères », encore appelés « isomères optiques ». Un atome de carbone lié à quatre substituants non identiques est appelé un « centre chiral ». Lorsqu'une molécule possède un tel centre chiral, elle est dite chirale et possède deux formes énantiomères. Lorsqu'une molécule possède plusieurs centres chiraux, alors elle possédera plusieurs formes diastéréoisomères et énantiomères. Un mélange équimolaire de deux énantiomères est appelé mélange racémique.

L'expression « composés de la présente invention » ou « composés de formule (I) » ou « composé (I) » telle qu'utilisée dans la présente description désigne des composés de formule (I), mais également de sous-formules (Ia) et (Ib) tels que définis de manière détaillée ci-dessous, et leurs sels pharmaceutiquement acceptables.

### DESCRIPTION DETAILLEE DE L'INVENTION

### Composés de formule (I) selon l'invention

L'invention a pour objet les composés de formule (I) telle que définie ci-dessus ou un sel pharmaceutiquement acceptable de ceux-ci.

Selon un premier mode de réalisation X représente un azote.

Selon un second mode de réalisation X représente un groupe CH.

Avantageusement, dans ces deux modes de réalisation, selon une première variante, R³ représente un groupe (C₁-C₆)alkyle, de préférence un groupe méthyle.

En particulier, dans ces deux modes de réalisation et éventuellement selon la première variante, selon une deuxième variante, R² représente un groupe (C₁-C₆)alkyle, de préférence un groupe méthyle, ou un groupe aryle-(C₁-C₆)alkyle, de préférence un groupe benzyle. Plus particulièrement, R² représente un groupe (C₁-C₆)alkyle, de préférence un groupe méthyle.

De préférence, dans ces deux modes de réalisation et leurs variantes, R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle.

En particulier, R⁴¹, R⁴², R⁴³ et R⁴⁴ représentent indépendamment les uns des autres un atome d'hydrogène, -OR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène, ou un groupe (C₁-C₆)alkyle.

Selon un mode de réalisation particulier, R⁴¹, R⁴², R⁴³ et R⁴⁴ représentent un atome d'hydrogène.

De préférence, dans ces deux modes de réalisation et leurs variantes, R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle, à condition que lorsque X représente un atome d'azote, R¹ représente un atome de chlore, un groupe -CN ou un groupe -CF₃, ou R⁵ et R⁶ forment ensemble une chaine -CR⁵¹=CR⁵²-CR⁵³=CR⁵⁴-.

Alternativement, dans ces deux modes de réalisation et leurs variantes, R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle, à condition que lorsque X représente un atome d'azote, R¹ représente un atome de chlore, un groupe -CN ou un groupe -CF₃, ou R⁵ et R⁶ forment ensemble une chaine -CR⁵¹=CR⁵²-CR⁵³=CR⁵⁴-.

En particulier, R⁵ et R⁶ représentent un atome d'hydrogène ou forment ensemble une chaine - CR⁵¹=CR⁵²-CR⁵³=CR⁵⁴- avec R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs groupes -OR⁵⁵, avec R⁵⁵ et R⁵⁶ représentant indépendamment l'un de l'autre un atome d'hydrogène ou un groupe (C₁-C₆)alkyle, à condition que lorsque X représente un atome d'azote, R¹ représente un atome de chlore, un groupe -CN ou un groupe -CF₃, ou R⁵ et R⁶ forment ensemble une chaine -CR⁵¹=CR⁵²-CR⁵³=CR⁵⁴-.

De préférence, R⁵ et R⁶ représentent un atome d'hydrogène, à condition que lorsque X représente un atome d'azote, R¹ représente un atome de chlore, un groupe -CN ou un groupe -CF₃.

Alternativement, dans ces deux modes de réalisation et leurs variantes, R⁵ et R⁶ forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, un atome d'halogène, -OR⁵⁵, -SR⁵⁵, -NR⁵⁵R⁵⁶, -NO₂ ou un groupe (C₁-C₆)alkyle éventuellement substitué par un ou plusieurs atomes d'halogène ou groupes -OR⁵⁵, -SR⁵⁵, -NR⁵⁵R⁵⁶ et -NO₂, avec R⁵⁵ et R⁵⁶ représentant indépendamment l'un de l'autre un atome d'hydrogène, un groupe (C₁-C₆)alkyle, un groupe -CF₃ ou un groupe -COR⁵⁷ avec R⁵⁷ représentant un groupe (C₁-C₆)alkyle ou un groupe aryle.

De préférence, R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

Alternativement, R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle.

De préférence, R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène ou un groupe (C₁-C₆)alkyle.

En particulier, dans ces deux modes de réalisation et leurs variantes :
- R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle, et
- R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle,
   à condition que lorsque X représente un atome d'azote, R¹ représente un atome de chlore, un groupe -CN ou un groupe -CF₃, ou R⁵ et R⁶ forment ensemble une chaine -CR⁵¹=CR⁵²-CR⁵³=CR⁵⁴-.

Plus particulièrement,
- R⁴¹, R⁴², R⁴³ et R⁴⁴ représentent indépendamment les uns des autres un atome d'hydrogène, - OR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène, ou un groupe (C₁-C₆)alkyle, et
- R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle,
   à condition que lorsque X représente un atome d'azote, R¹ représente un atome de chlore, un groupe -CN ou un groupe -CF₃, ou R⁵ et R⁶ forment ensemble une chaine -CR⁵¹=CR⁵²-CR⁵³=CR⁵⁴-.

Les composés de formule (I) pourront notamment être choisis parmi les composés suivants :

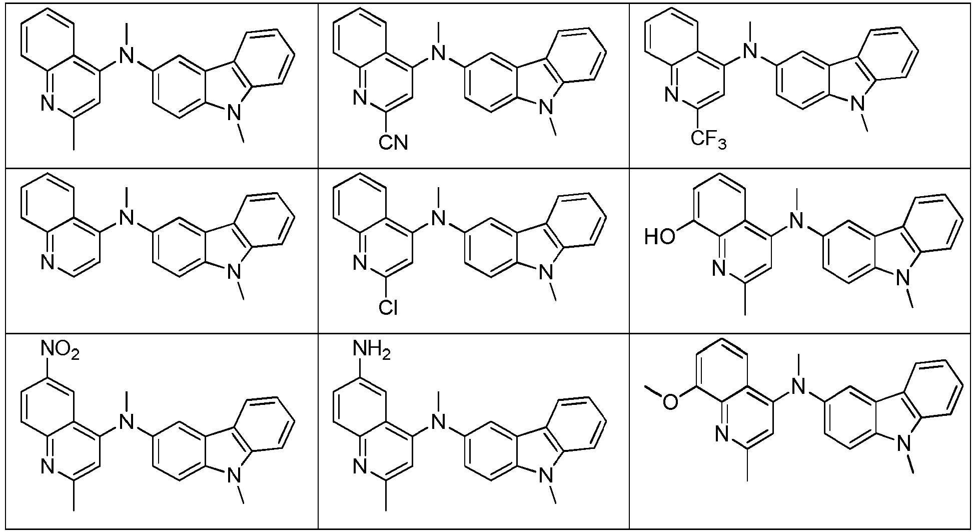

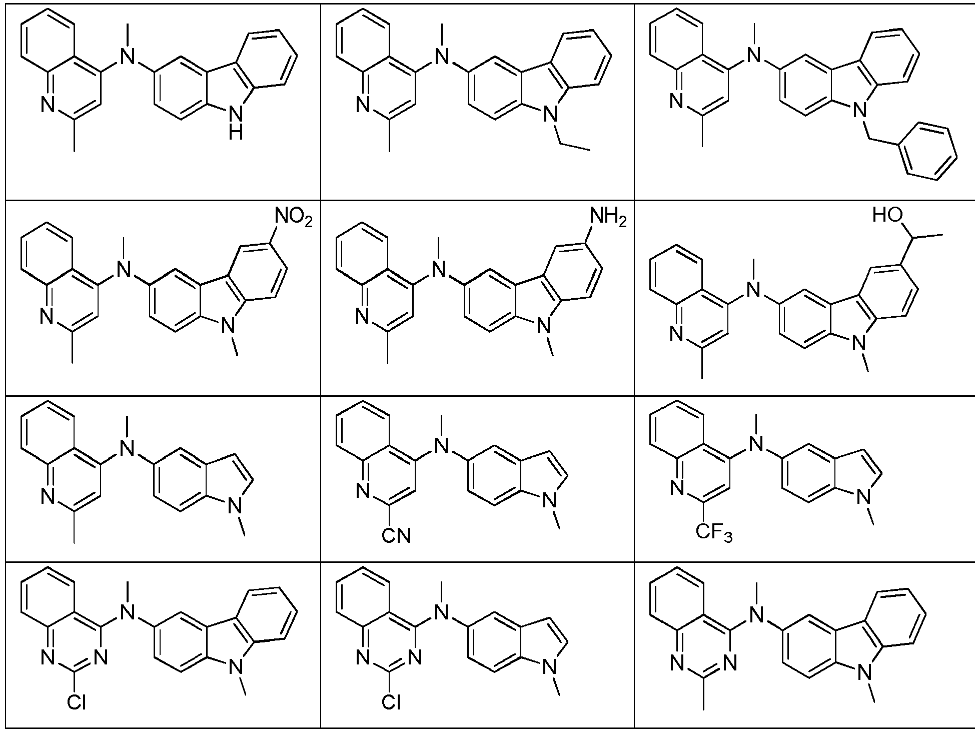

et leurs sels pharmaceutiquement acceptables.

### Composés de formule (la) selon l'invention

Selon un premier mode de réalisation particulier de l'invention, les composés de formule (I) selon l'invention sont des composés de formule (la) ci-dessous ou un sel pharmaceutiquement acceptable de ceux-ci : 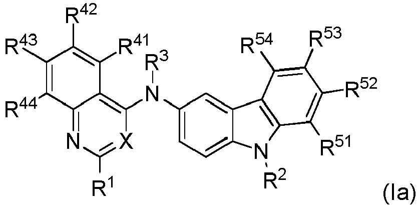 dans laquelle X, R¹, R², R³, R⁴¹, R⁴², R⁴³, R⁴⁴, R⁵¹, R⁵², R⁵³ et R⁵⁴ sont tels que définis ci-dessus.

Selon un premier mode de réalisation X représente un azote.

Selon un second mode de réalisation X représente un groupe CH.

Avantageusement, dans ces deux modes de réalisation, selon une première variante, R³ représente un groupe (C₁-C₆)alkyle, de préférence un groupe méthyle.

En particulier, dans ces deux modes de réalisation et éventuellement selon la première variante, selon une deuxième variante R² représente un groupe (C₁-C₆)alkyle, de préférence un groupe méthyle, ou un groupe aryle-(C₁-C₆)alkyle, de préférence un groupe benzyle. Plus particulièrement, R² représente un groupe (C₁-C₆)alkyle, de préférence un groupe méthyle.

De préférence, dans ces deux modes de réalisation et leurs variantes, R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle.

En particulier, R⁴¹, R⁴², R⁴³ et R⁴⁴ représentent indépendamment les uns des autres un atome d'hydrogène, -OR⁴⁵, -NR⁴⁵R⁴⁶ ou -NO₂ avec R⁴⁵ et R⁴⁶ représentant indépendamment l'un de l'autre un atome d'hydrogène, un groupe (C₁-C₆)alkyle.

Selon un mode de réalisation particulier, R⁴¹, R⁴², R⁴³ et R⁴⁴ représentent un atome d'hydrogène.

En particulier, dans ces deux modes de réalisation et leurs variantes, R⁵¹, R⁵², R⁵³ et R⁵⁴ représentant indépendamment les uns des autres un atome d'hydrogène, un atome d'halogène, -OR⁵⁵, -SR⁵⁵, -NR⁵⁵R⁵⁶, -NO₂ ou un groupe (C₁-C₆)alkyle éventuellement substitué par un ou plusieurs atomes d'halogène ou groupes -OR⁵⁵, -SR⁵⁵, -NR⁵⁵R⁵⁶ et -NO₂, avec R⁵⁵ et R⁵⁶ représentant indépendamment l'un de l'autre un atome d'hydrogène, un groupe (C₁-C₆)alkyle, un groupe -CF₃ ou un groupe -COR⁵⁷ avec R⁵⁷ représentant un groupe (C₁-C₆)alkyle ou un groupe aryle.

De préférence, R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

Alternativement, R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle.

De préférence, R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène ou un groupe (C₁-C₆)alkyle.

En particulier, dans ces deux modes de réalisation et leurs variantes :
- R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle, et
- R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

Plus particulièrement,
- R⁴¹, R⁴², R⁴³ et R⁴⁴ représentent indépendamment les uns des autres un atome d'hydrogène, - OR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène, ou un groupe (C₁-C₆)alkyle, et
- R⁵¹, R⁵², R⁵³ et R⁵⁴ représentant indépendamment les uns des autres un atome d'hydrogène, - OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle.

Les composés de formule (la) pourront notamment être choisis parmi les composés suivants :

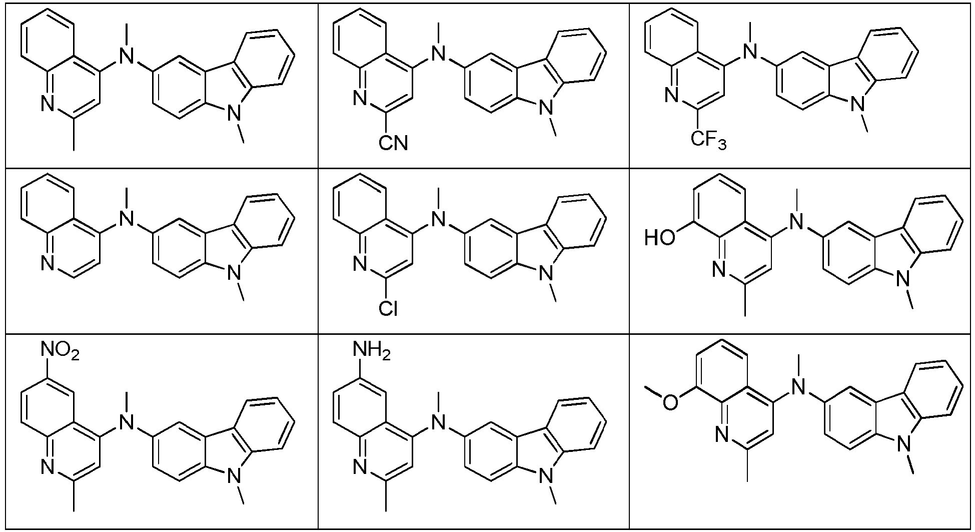

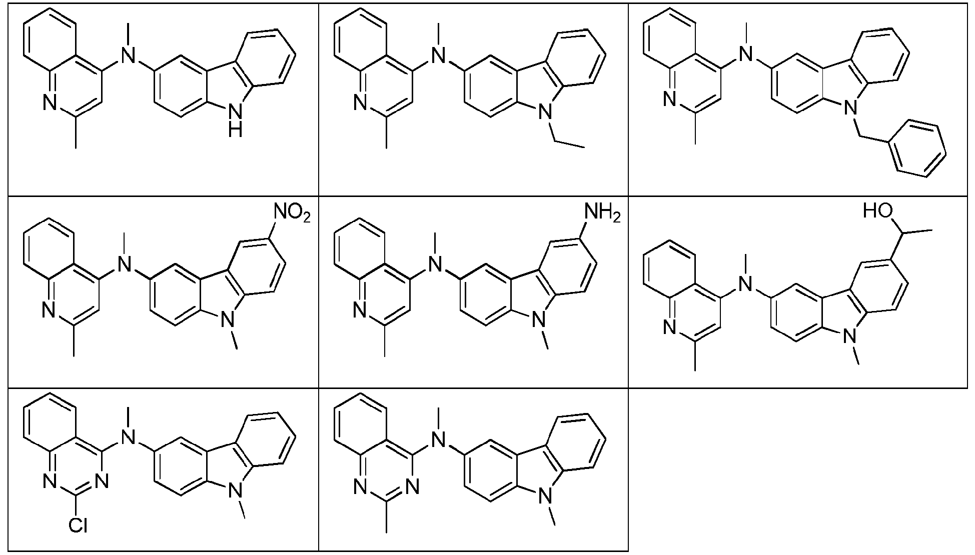

et leurs sels pharmaceutiquement acceptables.

### Composés substitués par un groupement R¹ électro-attracteur selon l'invention

Selon un deuxième mode de réalisation particulier de l'invention, les composés de formule (I) selon l'invention sont des composés de formule (I) telle que définie ci-dessus dans laquelle R¹ représente un atome de chlore, un groupe -CN ou un groupe -CF₃, ou un sel pharmaceutiquement acceptable de ceux-ci.

En particulier, R¹ représente un atome de chlore.

Selon un premier mode de réalisation X représente un azote.

Selon un second mode de réalisation X représente un groupe CH.

Avantageusement, dans ces deux modes de réalisation, selon une première variante, R³ représente un groupe (C₁-C₆)alkyle, de préférence un groupe méthyle.

En particulier, dans ces deux modes de réalisation et éventuellement selon la première variante, selon une deuxième variante, R² représente un groupe (C₁-C₆)alkyle, de préférence un groupe méthyle, ou un groupe aryle-(C₁-C₆)alkyle, de préférence un groupe benzyle. Plus particulièrement, R² représente un groupe (C₁-C₆)alkyle, de préférence un groupe méthyle.

De préférence, dans ces deux modes de réalisation et leurs variantes, R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle.

En particulier, R⁴¹, R⁴², R⁴³ et R⁴⁴ représentent indépendamment les uns des autres un atome d'hydrogène, -OR⁴⁵, -NR⁴⁵R⁴⁶ ou -NO₂ avec R⁴⁵ et R⁴⁶ représentant indépendamment l'un de l'autre un atome d'hydrogène, un groupe (C₁-C₆)alkyle. Plus préférentiellement, R⁴¹, R⁴², R⁴³ et R⁴⁴ représentent un atome d'hydrogène.

De préférence, dans ces deux modes de réalisation et leurs variantes, R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

Alternativement, dans ces deux modes de réalisation et leurs variantes, R⁵ et R⁶ représentent un atome d'hydrogène ou forment ensemble une chaine -CR⁵¹=CR⁵²-CR⁵³-CR⁵⁴- avec R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle. En particulier, R⁵ et R⁶ représentent un atome d'hydrogène ou forment ensemble une chaine - CR⁵¹=CR⁵²-CR⁵³=CR⁵⁴- avec R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs groupes -OR⁵⁵, avec R⁵⁵ et R⁵⁶ représentant indépendamment l'un de l'autre un atome d'hydrogène ou un groupe (C₁-C₆)alkyle.

De préférence, R⁵ et R⁶ représentent un atome d'hydrogène.

Alternativement, dans ces deux modes de réalisation et leurs variantes, R⁵ et R⁶ forment ensemble une chaine -CR⁵¹=CR⁵²-CR⁵³-CR⁵⁴- avec R⁵¹, R⁵², R⁵³ et R⁵⁴ représentant indépendamment les uns des autres un atome d'hydrogène, un atome d'halogène, -OR⁵⁵, -SR⁵⁵, -NR⁵⁵R⁵⁶, -NO₂ ou un groupe (C₁-C₆)alkyle éventuellement substitué par un ou plusieurs atomes d'halogène ou groupes -OR⁵⁵, -SR⁵⁵, -NR⁵⁵R⁵⁶ et -NO₂, avec R⁵⁵ et R⁵⁶ représentant indépendamment l'un de l'autre un atome d'hydrogène, un groupe (C₁-C₆)alkyle, un groupe -CF₃ ou un groupe -COR⁵⁷ avec R⁵⁷ représentant un groupe (C₁-C₆)alkyle ou un groupe aryle.

De préférence, R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

Alternativement, R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle.

De préférence, R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène ou un groupe (C₁-C₆)alkyle.

En particulier, dans ces deux modes de réalisation et leurs variantes:
- R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle, et
- R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

Plus particulièrement,
- R⁴¹, R⁴², R⁴³ et R⁴⁴ représentent indépendamment les uns des autres un atome d'hydrogène, - OR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène, ou un groupe (C₁-C₆)alkyle, et
- R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle.

Les composés substitués par un groupement R¹ électro-attracteur pourront notamment être choisis parmi les composés suivants :

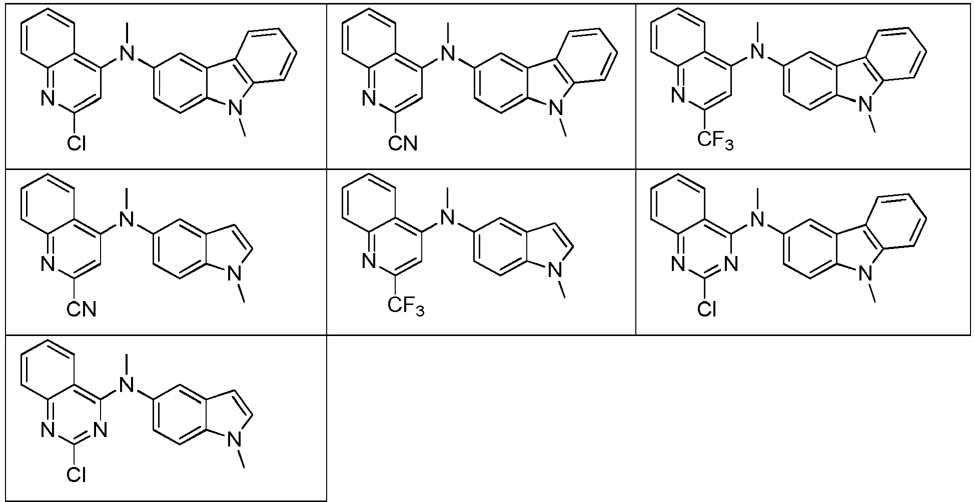

et leurs sels pharmaceutiquement acceptables.

En particulier, les composés substitués par un groupement R¹ électro-attracteur pourront être choisis parmi les composés suivants :

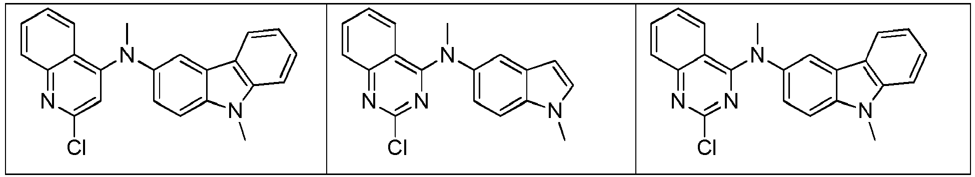

et leurs sels pharmaceutiquement acceptables.

### Composés de formule (Ib) selon l'invention

Selon un troisième mode de réalisation particulier de l'invention, les composés de formule (I) selon l'invention sont un composé de formule (Ib) 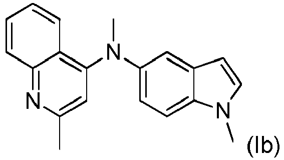 ou un sel pharmaceutiquement acceptable de celui-ci.

### Utilisations des composés selon l'invention

L'invention a pour objet les composés de formule (I), (la) ou (Ib) ainsi que leurs sels pharmaceutiquement acceptables, pour leur utilisation en tant que médicament.

En particulier, les composés de la présente invention peuvent être utilisés en tant que médicaments destinés à traiter le cancer.

La présente invention concerne également l'utilisation d'un composé de la présente invention pour la préparation d'un médicament destiné au traitement du cancer.

La présente invention concerne également une méthode de traitement du cancer comprenant l'administration à un patient en ayant besoin d'une quantité efficace d'un composé de la présente invention.

Les composés peuvent être utilisés seuls ou en association, avantageusement synergique, avec au moins un autre principe actif.

Parmi les cancers, on peut citer de manière non limitative les leucémies, les lymphomes, les sarcomes, le mélanome, le cancer du foie, le cancer du pancréas, le cancer du poumon, le cancer de l'estomac, le cancer de l'œsophage, le cancer du rein, le cancer de la plèvre, le cancer de la tyroïde, le cancer de la peau, le cancer du col de l'utérus le cancer du sein, le cancer des ovaires, le cancer colorectal, le cancer des testicules, le cancer de la prostate, le cancer du cerveau, le cancer du rectum, ou le cancer des os.

En particulier, le cancer sera choisi parmi les sarcomes, le mélanome, le cancer colorectal, le cancer du sein, le cancer des ovaires, le cancer du pancréas et les leucémies.

En particulier, le patient ayant besoin d'un traitement est un mammifère, notamment un humain.

Avantageusement, les composés selon l'invention peuvent être utilisés dans le traitement du cancer en agissant par inhibition de la polymérisation de la tubuline et/ou par immunomodulation. En effet, les composés selon l'invention peuvent présenter à la fois une activité antiproliférative, une activité antivasculaire, une activité d'inhibition de la polymérisation de la tubuline et une activité immunomodulatrice.

L'activité immunomodulatrice des composés selon l'invention consiste à activer le système immunitaire.

L'activité antiproliférative est observée à des concentrations variant du picomolaire au nanomolaire.

L'activité immunomodulatrice est observée à travers l'augmentation de la présentation antigénique via une prolifération lymphocytaire T CD8+ dans les cellules cancéreuses en présence des composés selon l'invention.

La présente invention concerne également une méthode pour inhiber la polymérisation de la tubuline et/ou activer l'immunomodulation comprenant l'administration à un patient en ayant besoin d'une quantité efficace d'un composé de la présente invention, seul ou en association, avantageusement synergique, avec au moins un autre principe actif, notamment tel que défini ci-dessus.

### Compositions pharmaceutiques selon l'invention

L'invention a également pour objet une composition pharmaceutique comprenant un composé de formule (I), (la), ou (Ib) selon l'invention, selon l'un quelconque des modes de réalisations décrits ci-dessus, et au moins un excipient pharmaceutiquement acceptable.

L'invention concerne également les compositions pharmaceutiques selon l'invention pour leur utilisation en tant que médicament, notamment destiné à traiter le cancer, en agissant en particulier par inhibition de la polymérisation de la tubuline et/ou par immunomodulation.

Les compositions pharmaceutiques selon l'invention peuvent être destinées à une administration par voie entérale (par exemple par voie orale) ou parentérale (par exemple intraveineuse), de préférence par voie orale ou par intraveineuse. L'ingrédient actif peut être administré sous des formes unitaires pour l'administration, mélangé avec des supports pharmaceutiques classiques, à des animaux, de préférence des mammifères, en particulier l'homme.

Pour l'administration orale, la composition pharmaceutique peut être sous une forme solide ou liquide (solution ou suspension).

Une composition solide peut se présenter sous la forme de comprimés, de gélules, de poudres, de granules et analogues. Dans les comprimés, l'ingrédient actif peut être mélangé avec un ou plusieurs véhicule(s) pharmaceutique(s) tel(s) que la gélatine, l'amidon, le lactose, le stéarate de magnésium, le talc, la gomme arabique et similaires, avant d'être comprimé. Les comprimés peuvent en outre être enrobés, notamment avec du saccharose ou avec d'autres matériaux appropriés, ou ils peuvent être traités de telle manière qu'ils aient une activité prolongée ou retardée. Dans les poudres ou les granules, l'ingrédient actif peut être mélangé ou granulé avec des agents dispersants, des agents mouillants ou des agents de mise en suspension et avec des correcteurs de saveur ou des édulcorants. Dans les gélules, l'ingrédient actif peut être introduit dans des gélules molles ou dures sous la forme d'une poudre ou de granules comme mentionné précédemment ou sous la forme d'une composition liquide comme mentionné ci-après.

Une composition liquide peut contenir l'ingrédient actif avec un édulcorant, un exhausteur de goût ou un colorant approprié dans un solvant tel que l'eau. La composition liquide peut également être obtenue en suspendant ou en dissolvant une poudre ou des granules, comme mentionné ci-dessus, dans un liquide tel que de l'eau, du jus, du lait, etc. Il peut s'agir par exemple d'un sirop ou d'un élixir.

Pour l'administration parentérale, la composition peut se présenter sous la forme d'une suspension aqueuse ou d'une solution qui peut contenir des agents de suspension et / ou des agents mouillants. La composition est avantageusement stérile. Elle peut se présenter sous la forme d'une solution isotonique (en particulier par rapport au sang).

Les composés selon l'invention peuvent être utilisés dans une composition pharmaceutique à une dose allant de 0,01 mg à 1000 mg par jour, administrés en une seule dose une fois par jour ou en plusieurs doses durant la journée, par exemple deux fois par jour à des doses égales. La dose administrée quotidiennement est avantageusement comprise entre 5 mg et 500 mg, et plus avantageusement entre 10 mg et 200 mg. Cependant, il peut être nécessaire d'utiliser des doses en dehors de ces plages, ce dont pourra se rendre compte l'homme du métier.

Selon un mode particulier de l'invention, les composés pour leur utilisation selon l'invention sont administrés en association avec un autre principe actif, notamment un composé anticancéreux, cytotoxique ou non. Ainsi, la composition pharmaceutique selon la présente invention peut comprendre en outre un autre principe actif.

Ainsi, la composition pharmaceutique selon l'invention comprend au moins un composé de la présente invention et au moins un autre principe actif en tant que produit de combinaison pour une utilisation simultanée, séparée ou étalée dans le temps, qui peut notamment être utilisée pour le traitement du cancer.

### Procédé de synthèse selon l'invention

L'invention a également pour objet un procédé de préparation d'un composé de formule (I), (la) ou (Ib) ou d'une sel pharmaceutiquement acceptable de celui-ci selon l'invention comprenant les étapes suivantes :
(1) couplage d'un dérivé aminé de formule (II) suivante : 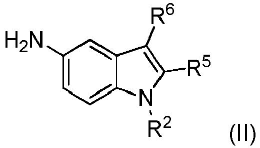 dans laquelle R², R⁵ et R⁶ sont tels que définis ci-dessus,
   avec un composé de formule (III) suivante : 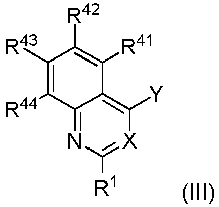 dans laquelle Y représente un atome de chlore, de brome, d'iode, un groupe trifluorométhylsulfonate ou un groupe tosyle et X, R¹, R⁴¹, R⁴², R⁴³ et R⁴⁴ sont tels que définis ci-dessus,
   pour donner un composé de formule (I), (la) ou (Ib) tel que défini ci-dessus avec R³ = H,
(2) éventuellement substitution du groupe R³ = H porté par l'amine du composé de formule (I), (la) ou (Ib) obtenu à l'étape (1) qui précède pour donner un composé de formule (I), (la) ou (Ib) tel que défini ci-dessus avec R³ représentant un groupe (C₁-C₆)alkyle, un groupe aryle-(C₁-C₆)alkyle ou un groupe -COR³¹ avec R³¹ tel que défini ci-dessus, et
(3) éventuellement salification du composé de formule (I) obtenu à l'étape (1) ou (2) qui précède pour donner un sel pharmaceutiquement acceptable d'un composé de formule (I) tel que défini ci-dessus.

Le couplage du dérivé aminé de formule (II) avec le composé de formule (III) peut être réalisé par substitution nucléophile aromatique, de préférence en milieu acide, notamment en présence d'acide chlorhydrique.

De préférence, le couplage est réalisé dans un solvant polaire aprotique, plus préférentiellement, le dioxane.

Avantageusement le couplage est réalisé à une température comprise entre 100 et 110°C, notamment à reflux du solvant.

Le couplage du dérivé aminé de formule (II) avec le composé de formule (III) peut également être réalisé par couplage pallado-catalysé en présence d'un complexe de palladium (0) ou d'un complexe de palladium (II) et d'une phosphine et en présence d'une base.

L'étape 2 de substitution peut être une étape d'alkylation lorsque R³ représente un groupe (C₁-C₆)alkyle. L'alkylation d'une amine est une réaction bien connue de l'homme du métier qui connaît les conditions réactionnelles à mettre en œuvre.

Elle peut être réalisée par exemple par réaction de l'amine avec un halogénure de (C₁-C₆)alkyle en présence d'une base, en particulier un carbonate. De préférence l'halogénure de (C₁-C₆)alkyle est un iodure de (C₁-C₆)alkyle, un bromure de (C₁-C₆)alkyle ou un chlorure de (C₁-C₆)alkyle. En particulier, l'alkylation est réalisée dans un solvant polaire aprotique, plus préférentiellement, le diméthylformamide. De préférence, l'alkylation a lieu à température ambiante.

L'étape 2 de substitution peut être une étape d'aryl-alkylation lorsque R³ représente un groupe aryle-(C₁-C₆)alkyle. L'aryle-alkylation d'une amine est une réaction bien connue de l'homme du métier qui connaît les conditions réactionnelles à mettre en œuvre. Elle peut être réalisée dans les mêmes conditions que pour la réaction d'alkylation décrite précédemment avec un halogénure d'aryle-(C₁-C₆)alkyle à la place de l'halogénure de (C₁-C₆)alkyle.

L'étape 2 de substitution peut être une étape de couplage peptidique lorsque R³ représente un groupe -COR³¹. Le couplage peptidique d'une amine est une réaction bien connue de l'homme du métier qui connaît les conditions réactionnelles à mettre en œuvre. Le couplage peptidique peut être réalisé avec un halogénure d'acyle (Hal-COR³¹ avec Hal = halogène), tel qu'un chlorure d'acyle (CICOR³¹), en présence d'une base, en particulier la triéthylamine. Le couplage peptidique peut être réalisé avec un acide carboxylique (R³¹COOH). Dans ce cas, celui-ci sera réalisé de préférence en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tetraméthyluronium hexafluorophosphate (HBTU), le 2-(IH-benzotriazole-1-yl)-1,1,3,3-tetraméthyluronium tetrafluoroborate (TBTU), le O-(7-azobenzotriazol-1-yl)-1,1,3,3-tétraméthyluronium hexafluorophosphate (HATU), le (benzotriazol-1-yloxy)tripyrrolodinophosphonium hexafluorophosphate (PyBOP) ou l'anhydride propylphosphonique, éventuellement associé à un auxiliaire de couplage tel que le N-hydroxy succinimide (NHS), le N-hydroxy benzotriazole (HOBt), le 3,4-dihydro-3-hydroxy-4-oxo-1,2,3-benzotriazole (HOBt), le I-hydroxy-7-azabenzotriazole (HOAt), le N-hydroxysylfosuccinimide (sulfo NHS), la diméthylaminopyridine (DMAP), la diisopropyléthylamine (DIEA) ou la N-méthylmorpholine (NMM). De préférence, il a lieu en présence de diméthylaminopyridine.

Le procédé de préparation d'un composé de formule (I), (la) ou (Ib) selon l'invention peut éventuellement comprendre une étape de salification du composé de formule (I), (la) ou (Ib) obtenu à l'étape (1) ou (2) pour donner un sel pharmaceutiquement acceptable d'un composé de formule (I), (la) ou (Ib). Cela implique la réaction du composé de formule (I), (la) ou (Ib) avec un acide ou une base pharmaceutiquement acceptable.

### EXEMPLES

### 1. Synthèse - Procédure expérimentale et caractérisation des produits

Dans un tube scellé sont ajoutés successivement le composé hétérocyclique chloré et le dérivé aromatique aminé dans le dioxane (2 mL). Une goutte d'HCI est ensuite ajoutée au mélange et le milieu réactionnel est chauffé à 100 °C, sous agitation, pendant 12 h. Le mélange est refroidi, puis neutralisé avec NaOH_{aq}. (5N) et la mixture est extraite à l'acétate d'éthyle (3 x 10 mL). Les phases organiques réunies sont séchées sur Na₂SO₄ et concentrées sous vide. Le brut réactionnel est dissout dans une solution de DMF (5 mL) contenant du Cs₂CO₃ (1.2 équiv.) à 0 °C. À ce mélange est ajouté au goutte-à-goutte CH₃I (1.2 équiv.) et le milieu réactionnel est placé à température ambiante, sous agitation, pendant 12 h. Le brut réactionnel est concentré et purifié par chromatographie sur colonne de silice.

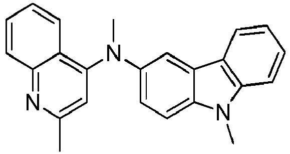

*N,9-Dimethyl-N-(2-methylquinolin-4-yl)-9H-carbazol-3-amine* **1** (24%). Solide jaune, F = 197.9 -198.2 °C. ¹H NMR (300 MHz, CDCl₃) δ 8.00 (d, *J* = 3.2 Hz, 1H), 7.97 (d, *J* = 3.8 Hz, 1H), 7.80 (d, *J* = 2.1 Hz, 1H), 7.61 (d, *J* = 8.5 Hz, 1H), 7.49 (t, *J* = 7.3 Hz, 2H), 7.39 (d, *J* = 8.2 Hz, 1H), 7.33-7.25 (m, 1H), 7.22 (d, *J* = 7.1 Hz, 1H), 7.17 (dd, *J* = 8.7 Hz, *J* = 2.1 Hz, 1H), 7.05 (t, *J* = 7.3 Hz, 1H), 6.97 (s, 1H), 3.82 (s, 3H), 3.57 (s, 3H), 2.77 (s, 3H). ¹³C NMR (75 MHz, CDCl₃) δ 159.4, 154.4, 149.8, 143.5, 141.7, 138.0, 129.0, 128.7, 126.1, 125.3, 124.1, 123.6, 122.5, 122.2, 121.8, 120.6, 118.9, 114.8, 110.9, 109.3, 108.7, 44.2, 29.3, 25.8. IR film vₘₐₓ/ cm⁻¹ : 1586, 1509, 1485, 1470, 1413, 1387, 1357, 1333, 1289, 1243, 1179, 1153, 1122, 1102, 1060.

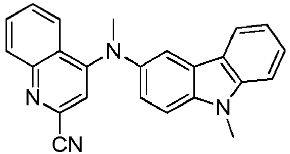

*4-*(*Methyl*(*9-methyl-9H-carbazol-3-yl*)*amino*)*quinoline-2-carbonitrile* **2** (28%). Solide jaune pâle. F = 232.9 -233.8 °C. ¹H NMR (300 MHz, CDCl₃) δ 7.92 (t, *J* = 8.2 Hz, 2H), 7.75 (d, *J* = 2.2 Hz, 1H), 7.51 - 7.38 (m, 3H), 7.34 (d, *J* = 8.2 Hz, 1H), 7.26 (d, *J* = 8.2 Hz, 1H), 7.22 - 7.07 (m, 3H), 7.06 - 6.97 (td, *J* = 8.2 Hz, *J* = 2.2 Hz, 1H), 3.79 (s, 3H), 3.52 (s, 3H). ¹³C NMR (75 MHz, CDCl₃) δ 154.7, 149.9, 142.2, 141.7, 138.7, 134.2, 130.3, 129.8, 126.6, 126.4, 125.7, 123.7, 122.9, 122.7, 122.2, 120.5, 119.2, 118.3, 116.3, 110.6, 109.7, 108.8, 44.8, 29.3. IR neat v_{max/}cm⁻¹: 2962, 2928, 2885, 2361, 2341, 1711, 1648, 1601, 1542, 1484, 1390, 1288, 1220, 1155, 1060, 972.

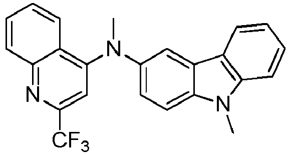

*N,9-Dimethyl-N-*(*2-*(*trifluoromethyl*)*quinolin-4-yl*)*-9H-carbazol-3-amine* **3** (26%). Solide jaune pâle. F = 190.3 - 192.0 °C. ¹H NMR (300 MHz, CDCl₃) δ 8.06 (dd, *J* = 7.8 Hz, *J* = 2.2 Hz, 1H), 7.94 (d, *J* = 7.8 Hz, 1H), 7.79 (d, *J* = 2.2 Hz, 1H), 7.58 - 7.41 (m, 3H), 7.36 (d, *J* = 8.2 Hz, 1H), 7.33 - 7.23 (m, 2H), 7.24 - 7.10 (m, 2H), 7.04 (td, *J* = 7.8 Hz, *J* = 6.8 Hz, 1H), 3.81 (s, 3H), 3.59 (s, 3H). ¹³C NMR (75 MHz, CDCl₃) δ 155.5, 149.1, 148.1 (q, *J* = 135 Hz), 142.7 (2), 141.6, 138.5, 130.4, 129.5, 126.3, 126.0, 125.6, 123.7, 122.8, 122.3, 122.0 (q, *J* = 1.1 kHz), 120.5, 119.1, 116.1, 109.6, 108.8, 104.0, 44.7, 29.2. ¹⁹F NMR (188 MHz, CDCl₃) δ -68.11. IR neat v_{max/}cm⁻¹ : 3052, 2961, 2918, 2851, 1585, 1570, 1494, 1426, 1400, 1171, 1142, 1124, 1104, 938, 733.

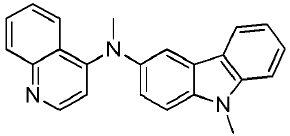

*N,9-Dimethyl-N-*(*quinolin-4-y*)*-9H-carbazol-3-amine* **4** (25%). Solide marron. F = 222.2 - 228.4 °C. ¹H NMR (300 MHz, DMSO) δ 8.84 (d, *J* = 8.5 Hz, 1H), 8.54 (d, *J* = 7.3 Hz, 1H), 8.32 - 8.26 (d, *J* = 2.8 Hz, 1H), 8.26 - 8.13 (m, 3H), 7.91 (t, *J* = 8.2 Hz, 1H), 7.82 (d, *J* = 8.5 Hz, 1H), 7.67 (d, *J* = 8.2 Hz, 1H), 7.54 (td, *J* = 5.7 Hz, *J* = 2.8 Hz, 2H), 7.25 (t, *J* = 7.3 Hz, 1H), 6.71 (d, *J* = 7.3 Hz, 1H), 4.17 (s, 3H), 3.96 (s, 3H). ¹³C NMR (75 MHz, DMSO) δ 155.5, 147.6, 141.3, 139.7, 138.8, 134.3, 128.1, 127.2, 126.5, 123.9, 123.8, 122.8, 121.6, 120.7, 119.2, 118.8, 118.0, 117.7, 110.5, 109.6, 99.7, 42.2, 29.3. IR neat v_{max/}cm⁻¹ : 3439, 3052, 2962, 2850, 1615, 1555, 1363, 1144, 1121, 1104, 1058, 746.

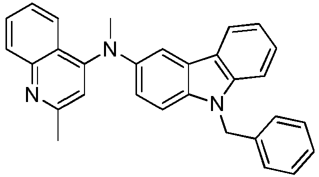

*9-Benzyl-N-methyl-N-*(*2-methylquinolin-4-yl*)*-9H-carbazol-3-amine* **5** (59%). Solide blanc. F = 192.7 - 196.5 °C. ¹H NMR (300 MHz, CDCl₃) δ 8.67 (d, *J* = 8.7 Hz, 1H), 8.06 (d, *J* = 7.7 Hz, 1H), 7.96 (s, 1H), 7.58 - 7.41 (m, 5H), 7.30 (s, 2H), 7.26 - 7.14 (m, 5H), 7.02 - 6.94 (t, *J* = *8.4* Hz, 1H), 6.80 (s, 1H), 5.57 (s, 2H), 3.80 (s, 3H), 3.03 (s, 3H).¹³C NMR (75 MHz, CDCl₃) δ 157.0, 155.3 (2), 141.6, 140.8, 139.1, 136.7, 131.6, 129.0 (2), 127.9, 127.1, 126.5 (2), 126.1, 125.4, 124.4, 123.1, 123.0, 122.5, 120.9, 120.0, 119.1, 116.9, 110.7, 109.6, 106.9, 47.0, 45.7, 21.8. IR neat v_{max/} cm⁻¹ : 3062, 2823, 2360, 2201, 1630, 1595, 1507, 1482, 1380, 906.

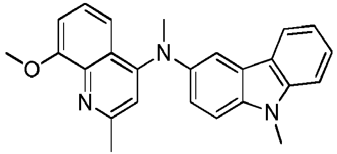

*N-(8-Methoxy-2-methylquinolin-4-yl)-N,9-dimethyl-9H-carbazol-3-amine* **6** (45%). Solide jaune. F = 137.6-143.3 °C. ¹H NMR (300 MHz, CDCl₃) 8.02 (d, *J* = 7.9 Hz, 1H), 7.86 (d, *J* = 2.0 Hz, 1H), 7.56 - 7.51 (m, 1H), 7.46 (d, *J* = 4.0 Hz, 1H), 7.41 (d, *J* = 4.0 Hz, 1H), 7.30 - 7.22 (m, 2H), 6.98 - 6.86 (m, 4H), 4.09 (s, 3H), 3.90 (s, 3H), 3.77 (s, 3H), 3.15 (s, 3H). ¹³C NMR (75 MHz, CDCl₃) δ 156.3, 156.0, 152.4, 141.8, 141.5, 138.9, 136.5, 126.6, 124.8, 123.8, 122.6, 122.3, 121.0, 120.7, 119.4, 117.5, 117.2, 116.0, 109.8, 109.3, 108.9, 108.9, 56.2, 45.4, 29.4, 24.2. IR neat v_{max/}cm⁻¹ : 2962, 2926, 2853, 1630, 1597, 1558, 1503, 1483, 1469, 1137, 1120, 1093, 1079, 747.

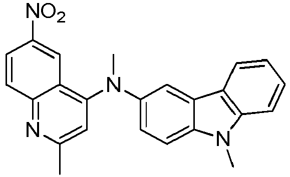

*N,9-Dimethyl-N-(2-methyl-6-nitroquinolin-4-yl)-9H-carbazol-3-amine* **7** (28%). Solide marron. F = 207.6 - 212.4 °C. ¹H NMR (300 MHz, CDCl₃) δ 8.52 (d, *J* = 2.4 Hz, 1H), 8.16 (dd, *J* = 7.4 Hz, *J* = 2.4 Hz, 1H), 7.93 (d, *J* = 6.7 Hz, 1H), 7.91 (d, *J* = 5.1 Hz, 1H), 7.71 (s, 1H), 7.51 - 7.32 (m, 4H), 7.17 (t, *J* = 7.4 Hz, 1H), 6.95 (s, 1H), 3.86 (s, 3H), 3.57 (s, 3H), 2.76 (s, 3H). ¹³C NMR (75 MHz, CDCl₃) δ 163.2, 155.6, 152.4, 142.9, 142.4, 141.7, 138.8, 130.2, 126.3, 123.8, 123.1, 123.0, 122.2, 122.1, 120.5, 120.0, 119.0, 116.2, 110.0 (2), 108.8, 44.5, 29.3, 26.0. IR film v_{max/} cm⁻¹ : 2963, 2930, 2854, 2361, 2341, 1735, 1633, 1613, 1522, 1335, 1049.

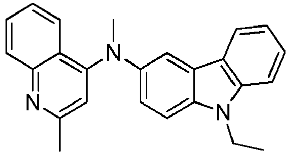

*9-Ethyl-N-methyl-N-(2-methylquinolin-4-yl)-9H-carbazol-3-amine* **8** (44%). Solide marron. F = 87.4 - 89.6 °C. ¹H NMR (300 MHz, CDCl₃) δ 8.55 (d, *J* = 8.7 Hz, 1H), 8.01 (d, *J* = 7.8 Hz, 1H), 7.91 (d, *J* = 2.2 Hz, 1H), 7.53 - 7.43 (m, 4H), 7.28 - 7.19 (m, 3H), 6.95 (t, *J* = 6.7 Hz, 1H), 6.86 (s, 1H), 4.40 (q, *J* = 7.1 Hz, 2H), 3.78 (s, 3H), 2.99 (s, 3H), 1.46 (t, *J* = 7.1 Hz, 3H). ¹³C NMR (75 MHz, CDCl₃) δ 157.1, 154.2, 140.8, 140.7, 139.7, 138.5, 131.7, 126.7, 126.3, 125.3, 124.0, 123.0, 122.2, 121.3, 120.7, 119.4, 118.4, 117.2, 110.2, 109.0, 106.0, 46.3, 37.9, 20.9, 13.9. IR film v_{max/} cm⁻¹ : 2957, 2924, 2855, 2360, 2340, 1730, 1633, 1594, 1508, 1346, 1042.

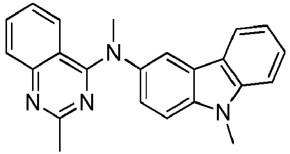

*N,9-Dimethyl-N-(2-methylquinazolin-4-yl)-9H-carbazol-3-amine* **9** (40%). Solide jaune. F = 181.5 - 183.6 °C. ¹H NMR (300 MHz, CDCl₃) δ 7.89 (d, *J* = 7.7 Hz, 1H), 7.84 (d, *J* = 2.1 Hz, 1H), 7.61 (d, *J* = 8.4 Hz, 1H), 7.39 (q, *J* = 6.8 Hz, 1H), 7.33 (s, 1H), 7.30 (d, *J* = 4.2 Hz, 1H), 7.21 - 7.08 (m, 3H), 6.81 (d, *J* = 8.4 Hz, 1H), 6.66 (t, *J* = 7.7 Hz, 1H), 3.77 (s, 3H), 3.61 (s, 3H), 2.64 (s, 3H). ¹³C NMR (75 MHz, CDCl₃) δ 163.3, 161.8, 141.7, 140.4 (2), 139.4, 134.0, 131.5, 127.5, 126.4 (2), 124.3, 123.8, 122.4, 120.6, 119.3, 118.1, 114.8, 109.7, 108.8, 43.4, 29.3, 26.5. IR neat v_{max/} cm⁻¹ : 3319, 2965, 2924, 2877, 1613, 1602, 1494, 1486, 1393, 1354, 1247.

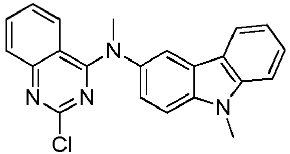

*N-(2-Chloroquinazolin-4-yl)-N,9-dimethyl-9H-carbazol-3-amine* **10** (62%). Solide jaune pâle. F = 231.2-232.1 °C. ¹H NMR (300 MHz, CDCl₃) δ 8.03 (d, *J* = 9.0 Hz, 1H), 7.98 (d, *J* = 2.1 Hz, 1H), 7.71 (d, *J* = 9.0 Hz, 1H), 7.60 - 7.40 (m, 4H), 7.34 - 7.27 (m, 2H), 6.80 (d, *J* = 4.1 Hz, 2H), 3.91 (s, 3H), 3.74 (s, 3H). ¹³C NMR (75 MHz, CDCl₃) δ 162.9, 156.8, 153.2, 141.9, 139.9, 138.9, 132.6, 127.7, 126.8, 126.7, 125.0, 124.3, 124.1, 122.4, 120.8, 119.6, 118.4, 115.1, 110.1, 109.1, 44.1, 29.5. IR neat v_{max/} cm⁻¹ : 3053, 2930, 1632, 1600, 1528, 1504, 1469, 1393, 927.

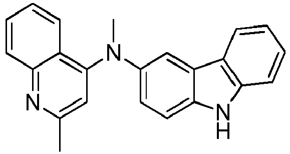

*N-Methyl-N-(2-methylquinolin-4-yl)-9H-carbazol-3-amine* **11** (12%). Dans un tube scellé sec et purgé à l'argon est ajouté **5** (85 mg, 0.2 mmol) dans l'acide trifluoroacétique (3 mL) avec 4 gouttes d'acide triflique. Le mélange est chauffé à 85 °C, sous agitation, pendant 4 h. Le milieu réactionnel est refroidi et le dichlorométhane (20 mL) est ajouté. La phase organique est lavée à l'eau (20 mL), séchée sur Na₂SO₄ and filtrée sur papier filtre. Le filtrat est concentré et purifié par chromatographie sur colonne de silice avec un mélange dichlorométhane / méthanol [95;5] et **11** est obtenu (8 mg, 12%). Solide jaune. F = 147.5 -149.2 °C. ¹H NMR (400 MHz, MeOD) δ 7.96 - 7.93 (m, 2H), 7.77 (d, *J* = 8.3 Hz, 1H), 7.57 (t, *J* = 7.4 Hz, 1H), 7.53 (d, *J* = 8.6 Hz, 1H), 7.46 (d, *J* = 8.1 Hz, 1H), 7.38 (t, *J* = 7.4 Hz, 1H), 7.28 - 7.20 (m, 2H), 7.12 (t, *J* = 7.4 Hz, 1H), 7.04 (s, 1H), 6.95 (t, *J* = 7.4 Hz, 1H), 3.73 (s, 3H), 2.76 (s, 3H). ¹³C NMR (101 MHz, MeOD) δ 158.9, 155.6, 142.4, 142.2, 141.2, 140.2, 133.1, 129.1, 128.1, 127.5, 126.0, 125.5, 124.2, 123.8, 121.7, 121.3, 120.2, 118.2, 113.5, 112.2, 106.8, 46.4, 20.9. IR neat v_{max/} cm⁻¹ : 3471, 3065, 3026, 2413, 2360, 1732, 1668, 1614, 1579, 1521, 1488, 1261, 1161, 1016.

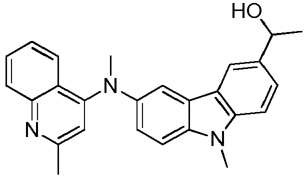

*1-(9-Methyl-6-(methyl(2-methylquinolin-4-yl)amino)-9H-carbazol-3-yl)ethanol* **12** (80%). Dans un bicol de 5 mL est ajouté **1** (40 mg, 0.11 mmol), AlCl₃ (15 mg, 0.11 mmol, 1 équiv.) et du dichlorométhane (3 mL). Le milieu réactionnel est chauffé à reflux et le chlorure d'acétyle (8 µL, 0.11 mmol, 1 équiv.) est ajouté goutte-à-goutte. Le milieu réactionnel est chauffé à reflux pendant 4 h. Le solvant est évaporé sous vide et un mélange dichlorométhane/eau [1;1] (1mL) y est ajouté. Du borohydrure de sodium NaBH₄ (7 mg, 0.11 mmol, 1équiv.) est ajouté doucement au milieu réactionnel, puis l'ensemble est placé à température ambiante et sous agitation pendant 4 h. L'eau est ajoutée doucement et la phase aqueuse est extraite au dichlorométhane. Les phases organiques sont rassemblées, séchées sur Na₂SO₄, puis filtrées sur papier filtre. Le filtrat est concentré et purifié par chromatographie sur colonne de silice avec un mélange dichlorométhane/méthanol [95 : 5] et **12** est obtenu (35 mg, 80%). Solide jaune. F = 152.4 -156.8 °C. ¹H NMR (300 MHz, MeOD) δ 8.05 (s, 2H), 7.82 (d, *J* = 8.3 Hz, 1H), 7.69 - 7.51 (m, 5H), 7.41 (dd, *J* = 8.8 Hz, *J* = 1.4 Hz, 1H), 7.34 (d, *J* = 8.5 Hz, 1H), 7.16 (s, 1H), 7.05 (t, *J* = 8.3 Hz, 1H), 5.02 (q, *J* = 6.6 Hz, 1H), 3.95 (s, 3H), 3.81 (s, 3H), 2.82 (s, 3H), 1.55 (d, *J* = 6.6 Hz, 3H). ¹³C NMR (75 MHz, MeOD) δ 158.5, 157.2, 146.8, 143.2, 142.5, 140.5, 138.4, 131.1, 127.3, 125.6, 125.5 125.3, 124.9, 124.0, 123.3, 121.2, 118.3, 117.2, 111.0, 109.7, 71.2, 49.0, 45.5, 29.5 (2), 25.9. IR neat v_{max/} cm⁻¹ : 3407, 2924, 2853, 2341, 1635, 1596, 1509, 1490, 1424, 1348, 1179, 1072, 1007.

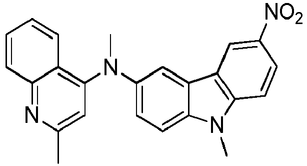

*N,9-dimethyl-N-(2-methylquinolin-4-yl)-6-nitro-9H-carbazol-3-amine* **13** (72%). À une solution de **1** (600 mg, 1.709 mmol) dans le dichlorométhane (30 mL) est ajouté goutte-à-goutte HNO₃ (69%, 111 µL, 1eq.) à 0 °C et sous argon. Après addition complète de HNO₃, le milieu réactionnel est laissé sous agitation et à température ambiante pendant 3 h. Le milieu est concentré, purifié par chromatographie sur colonne de silice avec un mélange dichlorométhane / méthanol [95;5], et **13** est obtenu (487 mg, 72%) après recristallisation dans un mélange cyclohexane / dichlorométhane [80;20]. Cristaux oranges. F = 182.4 - 183.4 °C. ¹H NMR (300 MHz, CDCl3) δ 7.92 (t, *J* = 8.0 Hz, 2H), 7.62 (d, *J* = 8.1 Hz, 1H), 7.58 (d, *J* = 7.3 Hz, 1H), 7.49 - 7.43 (m, 2H), 7.32 (t, *J* = 8.6 Hz, 2H), 7.03 (m, 2H), 6.93 (s, 1H), 3.85 (s, 3H), 3.46 (s, 3H), 2.74 (s, 3H). ¹³C NMR (75 MHz, CDCl₃) δ 159.1, 153.2, 149.7, 142.1, 140.9, 139.7, 134.5, 129.1, 128.9, 127.9, 124.6, 124.5, 124.3, 122.0, 121.0, 120.5, 118.6, 115.0, 111.8, 109.6, 109.3, 44.2, 29.5, 25.9. IR film vmax/ cm-1 : 2957, 2832, 2341, 1845, 1734, 1684, 1559, 1525, 1475, 1157, 682.

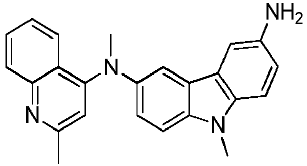

*N3,9-dimethyl-N3-(2-methylquinolin-4-yl)-9H-carbazole-3,6-diamine* **14** (87%). Dans un bicol de 25 mL est ajouté **13** (100 mg, 0.25 mmol) et le Pd/C (10%, 6 mg, 0.02eq.) dans l'éthanol (3mL). L'hydrazine hydratée est ajouté doucement au milieu réactionnel et l'ensemble est chauffé à reflux, sous agitation pendant 6 h. Le brut réactionnel est refroidi à température ambiante et filtré sur célite. Le filtrat est concentré et purifié par chromatographie sur colonne de silice avec un mélange dichlorométhane / éthanol [90;10] et **14** est obtenu (143 mg, 87%). Solide jaune. F = 137.8 - 138.3 °C. ¹H NMR (300 MHz, MeOD) δ 8.78 (d, *J* = 8.4 Hz, 1H), 8.04 (t, *J* = 7.6 Hz, 1H), 7.93 (d, *J* = 8.2 Hz, 1H), 7.85 (t, *J* = 7.6 Hz, 1H), 7.59 (d, *J* = 8.8 Hz, 1H), 7.55 (d, *J* = 8.2 Hz, 1H), 7.44 (d, *J* = 8.2 Hz, 1H), 7.35 (t, *J* = 7.5 Hz, 1H), 7.16 (d, *J* = 8.8 Hz, 1H), 6.85 (t, *J* = 7.6 Hz, 1H), 6.09 (s, 1H), 3.87 (s, 3H), 2.90 (s, 3H), 2.40 (s, 3H). ¹³C NMR (75 MHz, MeOD) δ 157.8, 155.9, 143.0, 141.3, 140.6, 136.4, 134.8, 128.0, 127.0, 124.3, 122.5, 121.5, 121.2, 121.0, 119.4, 117.9, 115.7, 112.7, 111.2, 109.8, 101.4, 31.8, 29.4, 20.4. IR film vmax/ cm-1 : 2924, 2853, 2341, 2327, 2273, 1845, 1734, 1635, 1603, 1558, 1521, 1474, 1438, 1365, 1338, 1090.

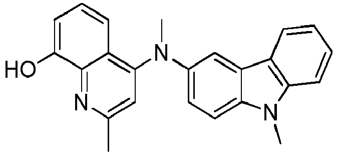

*2-Methyl-4-(methyl(9-methyl-9H-carbazol-3-yl)amino)quinolin-8-ol* **15.** À une solution de **6** (20 mg, 0.052 mmol) dans le dichlorométhane sec (1 mL) est ajouté goutte-à-goutte BBr₃(10 µL, 2eq.) à - 50 °C sous argon. Après addition complète de BBr₃, le milieu réactionnel est laissé sous agitation et à température ambiante pendant 2 h. Le pH du milieu réactionnel est ajusté à pH = 8 à l'aide d'une solution aqueuse de NaHCO₃, puis la phase organique est isolée et la phase aqueuse est extraite au dichlorométhane. Les phases organiques sont rassemblées et séchées sur Na₂SO₄, puis filtrées sur papier filtre. Le filtrat est concentré et purifié par chromatographie sur colonne de silice avec un mélange dichlorométhane / méthanol [95;5] et **15** est obtenu (17 mg, 87%). Solide marron. F = 222.4 - 223.4 °C. ¹H NMR (300 MHz, CDCl₃) δ 7.97 (d, *J* = 7.8 Hz, 1H), 7.80 (d, *J* = 2.1 Hz, 1H), 7.47 (t, *J* = 8.7 Hz, 1H), 7.39 (d, *J* = 8.2 Hz, 1H), 7.29 (d, *J* = 8.7 Hz, 1H), 7.22 - 7.15 (m, 2H), 6.97 - 6.84 (m, 4H), 3.84 (s, 3H), 3.55 (s, 3H), 2.70 (s, 3H). ¹³C NMR (75 MHz, CDCl₃) δ 157.1, 154.5, 152.0, 143.2, 141.7, 139.6, 138.3, 126.2, 124.6, 123.6 (2), 122.7, 121.5, 120.6, 119.0, 115.9, 115.4, 110.7, 109.4, 108.9, 108.8, 44.4, 29.4, 25.3. IR neat v_{max/} cm⁻¹ : 3051, 2878, 2360, 1564, 1511, 1484, 1471, 1424, 1357, 1293, 1243, 1083.

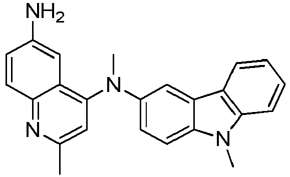

*N4,2-Dimethyl-N4-(9-methyl-9H-carbazol-3-yl)quinoline-4,6-diamine* **16.** Dans un bicol de 25 mL est ajouté **7** (180 mg, 0.45 mmol) à un mélange ethanol / eau [8;2]. Le milieu réactionnel est chauffé à 90°C jusqu'à solubilisation complète de **7.** Fe solide (254 mg, 4.5 mmol, 10 équiv.) et 3 gouttes d'HCI sont ajoutés au milieu réactionnel et l'ensemble est chauffé à 90 °C, sous agitation pendant 4 h. Le brut réactionnel est refroidi à température ambiante et filtré sur papier filtre. Le filtrat est concentré et purifié par chromatographie sur colonne de silice avec un mélange dichlorométhane / éthanol [95;5] et **16** est obtenu (143 mg, 87%). Solide jaune pâle. F = 192.6 - 196.5 °C. ¹H NMR (300 MHz, CDCl₃) δ 8.02 (d, *J* = 7.7 Hz, 2H), 7.81 (d, *J* = 2.1 Hz, 1H), 7.52 (t, *J* = 7.5 Hz, 1H), 7.42 (d, *J* = 8.2 Hz, 1H), 7.34 (d, *J* = 8.5 Hz, 1H), 7.24 (t, *J* = 7.5 Hz, 1H), 7.15 (dd, *J* = 7.7 Hz, *J* = 2.1 Hz, 1H), 6.98 (d, *J* = 11.1 Hz, 1H), 6.83 (s, 1H), 6.57 (s, 1H), 4.78 (s, 2H), 3.87 (s, 3H), 3.60 (s, 3H), 2.79 (s, 3H). ¹³C NMR (75 MHz, CDCl₃) δ 155.8, 153.0, 143.3, 142.9, 141.7, 137.6, 134.4, 130.2, 126.0, 123.6, 123.5, 122.6, 121.2, 120.8, 120.6, 118.8, 113.2, 112.9, 109.2, 108.7, 106.4, 43.7, 29.3, 25.3. IR neat v_{max/} cm⁻¹ : 2928, 2360, 2341, 1744, 1623, 1470, 1426, 1248, 1122.

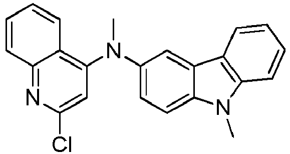

*N-(2-Chloroquinolin-4-yl)-N,9-dimethyl-9H-carbazol-3-amine* **17.** Dans un tube sec et purgé à l'argon sont ajoutés successivement le triflate de 2-hydroxyquinolin-4-yl (26 mg, 0.14 mmol, 1 équiv.), 9-methyl-9H-carbazol-3-amine (40 mg, 0.21 mmol, 1.5 équiv.), Pd₂dba₃ (8 mg, 6%), ± BINAP (11 mg, 12%), Cs₂CO₃ (112 mg, 2.5 équiv.) dans du dioxane sec (1.7 mL). Le milieu réactionnel est chauffé à 130°C, sous irradiation micro-onde pendant 1h 30. Le milieu réactionnel est refroidi à température ambiante, puis filtré sur Célite à l'aide un mélange dichlorométhane / méthanol [8 ; 2]. Le filtrat est concentré et POCl₃ (3 mL) y est ajouté doucement. La NEt₃ (0.5 mL) est additionnée goutte-à-goutte au milieu réactionnel, puis l'ensemble est chauffé à 90 °C, sous agitation pendant 4 h. Le POCl₃ est distillé sous vide et l'acétate d'éthyle (20 mL) est ajouté. La phase organique est lavée avec une solution de NaHCO₃, séchée sur Na₂SO₄ et filtrée sur papier filtre. Le filtrat est concentré et ajouté à une solution de Cs₂CO₃ (70 mg, 1.5 équiv.) dans du DMF (5mL) à 0° C. CH₃I (14µL, 1.5 équiv.) est ajouté goutte-à-goutte à 0°C au brut réactionnel qui est laissé à température ambiante, sous agitation pendant 12 h. Le brut réactionnel est concentré et purifié par chromatographie sur colonne de silice avec un mélange cyclohexane / acétate d'éthyle [90;10] et **17** est obtenu (4 mg, 8%). Solide jaune. F = 137.5 - 139.2°C. ¹H NMR (300 MHz, CDCl₃) δ 7.98 (d, *J* = 7.8 Hz, 1H), 7.89 (dd, *J* = 8.8 Hz, *J* = 1.4 Hz, 1H), 7.81 (d, *J* = 2.2 Hz, 1H), 7.51 - 7.43 (m, 3H), 7.40 (d, *J* = 8.2 Hz, 1H), 7.32 (d, *J* = 8.8 Hz, 1H), 7.24 - 7.16 (m, 2H), 7.02 - 6.96 (m, 2H), 3.85 (s, 3H), 3.55 (s, 3H). ¹³C NMR (75 MHz, CDCl₃) δ 156.1, 151.7, 149.4, 142.8, 141.8, 138.6, 129.6, 129.0, 126.4, 125.9, 124.9, 123.8, 122.9, 122.4, 121.7, 120.7, 119.2, 116.2, 109.6, 109.1, 108.9, 44.9, 29.4. IR neat v_{max/} cm⁻¹ : 2937, 2351, 2312, 1795, 1656, 1445, 1427, 1289, 1101.

***Procédure générale de synthèse des composés* 18-20**. Dans un tube scellé sec et purgé à l'argon sont ajoutés successivement le dérivé aromatique chloré, le 1-methyl-1H-indol-5-amine, Pd₂dba₃ (10 mol%), Xphos (20 mol%), NaO*t*Bu (3 équiv.) dans du toluène sec (2 mL). Le milieu réactionnel est chauffé à 100 °C, sous agitation pendant 12 h. Le milieu réactionnel est refroidi à température ambiante, filtré sur Célite. Le filtrat est concentré et ajouté à une solution de Cs₂CO₃ (70 mg, 1.5 équiv.) dans du DMF (5 mL) à 0 °C. CH₃I (14µL, 1.5 équiv.) est ajouté goutte-à-goutte à 0 °C au brut réactionnel et l'ensemble est laissé à température ambiante sous agitation pendant 12 h. Le brut réactionnel est concentré puis est purifié par chromatographie sur colonne de silice.

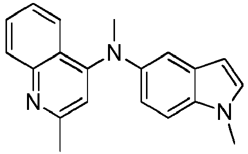

*N,2-Dimethyl-N-(1-methyl-1H-indol-5-yl)quinolin-4-amine* **18** (14%). Solide beige, F = 130.5 - 132.2 °C. ¹H NMR (300 MHz, CDCl₃) δ 7.94 (d, *J* = 8.3 Hz, 1H), 7.61 (d, *J* = 8.4 Hz, 1H), 7.48 (t, *J* = 7.6 Hz, 1H), 7.29 (d, *J* = 2.0 Hz, 1H), 7.22 (d, *J* = 8.7 Hz, 1H), 7.10 - 7.02 (m, 2H), 6.96 (dd, *J* = 8.7 Hz, *J* = 2.0 Hz, 1H), 6.91 (s, 1H), 6.38 (d, *J* = 3.0 Hz, 1H), 3.78 (s, 3H), 3.50 (s, 3H), 2.74 (s, 3H). ¹³C NMR (75 MHz, CDCl₃) δ 159.4, 154.6, 149.7, 144.0, 134.0, 129.9, 129.2, 128.9, 128.7, 125.4, 124.0, 121.9, 118.7, 115.0, 110.8, 110.2, 101.0, 44.2, 33.1, 25.8. IR neat v_{max/} cm⁻¹ : 1585, 1509, 1486, 1393, 1366, 1338, 1294, 1263, 1174, 1151, 1107, 1095, 1078, 1031.

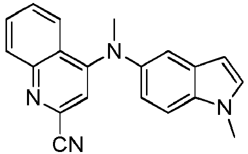

*04-(Methyl(1-methyl-1H-indo*/*-5-yl)amino)quinoline-2-carbonitrile* **19** (73%). Solide marron. F = 173.1 - 174.2 °C. ¹H NMR (300 MHz, CDCl₃) δ 7.99 (d, *J* = 8.4 Hz, 1H), 7.58 - 7.48 (m, 2H), 7.32 (d, *J* = 2.0 Hz, 1H), 7.29 (s, 1H), 7.18 (s, 1H), 7.15 - 7.07 (m, 2H), 6.96 (dd, *J* = 8.4, *J* = 2.0 Hz, 1H), 6.40 (d, *J* = 2.0 Hz, 1H), 3.80 (s, 3H), 3.53 (s, 3H). ¹³C NMR (75 MHz, CDCl₃) δ 155.0, 150.0, 142.8, 134.7, 134.2, 130.4, 130.3, 129.9, 129.3, 126.6, 125.9, 122.9, 119.2, 118.4, 116.7, 110.7, 110.6, 101.3, 44.8, 33.2. IR neat v_{max/} cm⁻¹: 2968, 2955, 2926, 2853, 2359, 1751, 1632, 1565, 1488, 1380, 1307, 1244, 1109, 1032, 909.

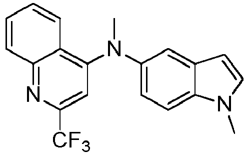

*N-Methyl-N-(1-methyl-1H-indol-5-yl)-2-(trifluoromethyl)quinolin-4-amine* **20** (62%). Solide jaune pâle. F = 108.5 - 109.5 °C. ¹H NMR (300 MHz, CDCl₃) δ 8.02 (d, *J* = 8.5 Hz, 1H), 7.54 - 7.43 (m, 2H), 7.27 (d, *J* = 2.0 Hz, 1H), 7.19 (d, *J* = 7.6 Hz, 2H), 7.09 - 6.99 (m, 2H), 6.91 (dd, *J* = 8.5 Hz, *J* = 2.0 Hz, 1H), 6.33 (d, *J* = 3.1 Hz, 1H), 3.73 (s, 3H), 3.49 (s, 3H). ¹³C NMR (75 MHz, CDCl₃) δ 155.7, 149.0, 148.4 (q, *J =* 33 Hz), 143.2, 134.4, 130.3, 130.1, 129.4, 129.1, 125.9, 125.7, 122.9, 122.0 (q, *J* = 273 Hz), 119.0, 116.3, 110.5, 103.8, 101.1, 44.6, 33.0. ¹⁹F NMR (188 MHz, CDCl₃) δ - 67.84. IR neat v_{max/} cm⁻¹ : 2963, 2926, 2853, 1598, 1569, 1513, 1488, 1365, 1189, 1154, 1128, 1111, 1093.

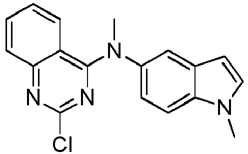

*2-Chloro-N-methyl-N-(1-methyl-1H-indol-5-yl)quinazolin-4-amine* **21.** Dans un tube scellé sec et purgé à l'argon sont ajoutés successivement la 2,4-dichloroquinazoline (139 mg, 0.70 mmol, 1 équiv), le 1-methyl-1*H*-indol-5-amine (83 mg, 0.84 mmol, 1.2 équiv) et du *t*-BuOK (102 mg, 0.91 mmol, 1.3 équiv) dans un mélange THF/eau [2:1] (10 mL). Le milieu réactionnel est agité à température ambiante pendant 48 h. H₂O (40 mL) est ajoutée au milieu réactionnel et le mélange est extrait à l'acétate d'éthyle. Les phases organiques sont rassemblées, séchées sur Na₂SO₄ et filtrées sur papier filtre. Le filtrat est concentré sous vide et est ajouté à une solution de NaH (84 mg, 3.5 mmol, 5 équiv) dans du DMF (5mL) à 0 °C. De l'iodure de méthyle (CH₃I, 218 µL, 2.5 mmol, 5 équiv) est ajouté goutte-à-goutte à 0 °C au le milieu réactionnel qui est ensuite laissé à température ambiante, sous agitation, pendant 12 h. Le brut réactionnel est concentré et purifié par chromatographie sur colonne de silice avec un mélange Dichlorométhane / Méthanol [90 :10] et le composé **21** est obtenu (16 mg, 10%). Solide blanc, F = 173.2-176.6 °C. ¹H NMR (300 MHz, CDCl₃) δ 8.11 (d, *J* = 7.7 Hz, 1H), 8.05 (d, *J* = 1.9 Hz, 1H), 8.02 (d, *J* = 9.3 Hz, 1H), 7.88 (d, *J* = 8.1 Hz, 1H), 7.65 (t, *J* = 7.6 Hz, 1H), 7.57 (t, *J* = 7.6 Hz, 1H), 7.48 (t, *J* = 9.3 Hz, 2H), 7.41 (dd, *J* = 8.6 Hz, *J* = 2.0 Hz, 1H), 7.32 (m, 2H), 6.76 (s, 1H), 3.91 (s, 3H), 3.75 (s, 3H). ¹³C NMR (75 MHz, CDCl₃) δ 157.7, 148.8, 142.1, 141.7, 139.7, 137.5, 130.5, 126.9, 126.4, 125.5, 124.1, 122.8, 122.5, 121.4, 120.6, 119.5, 119.4, 111.5, 110.0, 108.9, 39.6, 29.3. IR neat vₘₐₓ/ cm⁻¹ : 1565, 1503, 1488, 1445, 1423, 1399, 1365, 1334, 1301, 1281, 1268, 1243, 1208, 1168, 1093, 1013.

### 2. Etudes biologiques

### 2.1. Etude in vitro de la cytotoxicité

Les effets des composés selon l'invention sur la prolifération de différentes cellules cancéreuses ainsi que sur la prolifération de cellules endothéliales ont été étudiés.

L'activité biologique des composés de l'invention a été étudiée in vitro sur 5 lignées cellulaires cancéreuses humaines d'origines tissulaires différentes HCT116 carcinome colorectal ; A2780R cancer de l'ovaire Cisplatine résistant ; MiaPaca2 cancer du pancréas ; K562R leucémie myéloïde chronique doxorubicine résistante; JIM-T1 carcinome mammaire Trastuzumab-DM1 résistant; et HepG2 carcinome hépatique. Les cellules sélectionnées pour cette étude ont été incubées à 37 °C en présence de l'un des composés ajouté dans le milieu de culture à différentes concentrations. L'ensemble des expériences réalisées a permis de déterminer le degré de toxicité du composé testé, son effet sur le déroulement du cycle cellulaire ainsi que sa capacité à induire une mort cellulaire par apoptose.

Toutes les lignées cellulaires ont été maintenues en culture à 37 °C dans une atmosphère humide contenant 5% de CO₂. La viabilité cellulaire a été évaluée en utilisant le réactif CellTiter-Blue^{™} (Promega, WI, USA) en respectant les instructions du fabricant. Les cellules ont été ensemencées dans des plaques de culture de 96 puits à raison de 5000 cellules par 15 puits dans 50 µl de milieu de culture. Après 24 heures de culture, les composés de formule générale (I) dissous dans du DMSO ont été ajoutés individuellement dans chacun des puits à raison de 50 µl par puits. Tous les composés ont été testés en triplicat pour chaque concentration définie et chaque expérience a été répétée 3 fois. Après 72 heures d'incubation, 20 µL de resazurin ont été ajoutés dans chaque puits. Après 2 heures d'incubation, la fluorescence émise a été mesurée à 590 nm après excitation à 560 nm à l'aide d'un lecteur de fluorescence de type Victor (Perkin-Elmer, USA). La concentration de chacun des composés qui induit la mort de 50% des cellules (IC₅₀) a été déterminée après 72 heures d'incubation.

Les résultats sont reportés dans le tableau 1 ci-dessous et dans le tableau 2 dans le paragraphe suivant 2.2.

**Tableau 1 : Résultats de cytotoxicité des composés 1 et 21 sur six lignées cellulaires cancéreuses humaines.**

| Composé | IC₅₀ (nM) | | | | |
|---|---|---|---|---|---|
| | HCT116 | A2780R | MiaPaca2 | K562R | JIM-T1 |
| **1** | 0.07 ± 0.09 | 0.54 ± 0.094 | 0.47 ± 0.010 | 0.277 ± 0.017 | 0.36 ± 0.020 |
| **21** | 0.20 ± 0.01 | 2.34 ± 0.180 | 0.62 ± 0.016 | 1.77 ± 0.162 | 0.52 ± 0.016 |

### 2.2. Inhibition de la polymérisation de la tubuline

La tubuline est purifiée à partir de cervelles de brebis selon la méthode de Shelanski par 2 cycles d'assemblage-désassemblage. La solution mère (15-20 mg/mL), stockée à -196 °C, est décongelée et diluée dans le tampon d'assemblage (0.1 M MES, 0.5 mM MgCl₂, 1 mM EGTA, and 1 mM GTP, pH 6.6) pour avoir une concentration finale de 10 µM. L'assemblage de la tubuline est suivi par fluorescence sur plaques 96-puits selon la méthode de Barron et al. (Anal. Biochem. 315 (2003) 49-56). A la solution de tubuline (10 µM, 100 µL par puits) est ajouté l'inhibiteur (DMSO, 1 µL) et la solution est incubée 45 min à température ambiante. Le GTP (1 mM final) est ensuite ajouté, la solution est mélangée rapidement et la fluorescence (λex = 350 nm, λem = 440 nm) est mesurée sur un fluorimètre Wallac Victor (Perkin Elmer). La détermination de l'inhibition de 50% du taux d'assemblage maximum (IC₅₀) est réalisée en duplicate ou triplicate sur 10 concentrations encadrant la IC₅₀.

Les résultats sont reportés dans le tableau 2 ci-dessous.

**Tableau 2 : Résultats de cytotoxicité sur la lignée HCT116 et d'inhibition de la polymérisation de la tubuline (IPT) des composés selon l'invention.**

| Composé | **1** | **2** | **6** | **15** |
|---|---|---|---|---|
| IC₅₀ (HCT116) (nM) | 0.07 ± 0.09 | 12 ± 0.8 | 21.6 ± 0.71 | 50.6 ± 0.66 |
| IC₅₀ (IPT) (µM) | 1.13 ± 0.16 | 1.40 ± 0.51 | 1.48 ± 0.52 | 1.24 ± 0.40 |

| Composé | **16** | **17** | **18** | **19** |
|---|---|---|---|---|
| IC₅₀ (HCT116) (nM) | 24.5 ± 1.92 | 16.7 ± 0.38 | 2.00 ± 0.04 | 0.72 ± 0.03 |
| IC₅₀ (IPT) (µM) | 5.76 ± 1.47 | 1.16 ± 0.31 | 1.34 ± 0.45 | 2.78 ± 1.14 |

| Composé | **20** | **9** | **10** | **21** |
|---|---|---|---|---|
| IC₅₀ (HCT116) (nM) | 3.28 ± 0.18 | 2.4 ± 0.2 | 1.06 ± 0.2 | 0.20 ± 0.01 |
| IC₅₀ (IPT) (µM) | 2.50 ± 0.54 | 3.8 ± 0.9 | 3.14 ± 1.04 | 1.19 ± 0.28 |

| Composé | **14** | | | |
|---|---|---|---|---|
| IC₅₀ (HCT116) (nM) | 2.88 ± 1.54 | | | |
| IC₅₀ (IPT) (µM) | 2.76 ± 0.5 | | | |

### 2.3. Analyse du cycle cellulaire

Les cellules HCT116 sont ensemencées dans des plaques de culture 6 puits à raison de 300 000 cellules par puits dans leurs milieux respectifs décrits ci-dessus. Après 24 heures de culture, le composé **1** a été ajouté dans chacun des puits à différentes concentrations. Après 24 heures d'incubation, les cellules sont collectées individuellement dans des tubes de 15 mL puis centrifugées. Les cellules sont ensuite lavées 2 fois dans du PBS froid puis remises en suspension dans 1 mL de PBS, fixées en ajoutant 2 mL d'éthanol absolu froid et placées à 4°C pendant 1 heure. Après centrifugation, les cellules sont lavées 2 fois dans du PBS puis le culot cellulaire est repris dans 100 µL de Triton X100 à 1%. Après 30 minutes d'incubation à température ambiante, 50 µL de RNase A préalablement bouillies (1 mg/mL) et 500 µL d'iodure de propidium (50 µg/mL) sont ajoutés dans chaque tube et incubés dans l'obscurité à température ambiante pendant 30 minutes. La distribution du nombre de cellules dans chacune des phases du cycle cellulaire est ensuite déterminée par cytométrie en flux à l'aide d'un cytomètre de type FC500 (Beckman-Coulter, France).

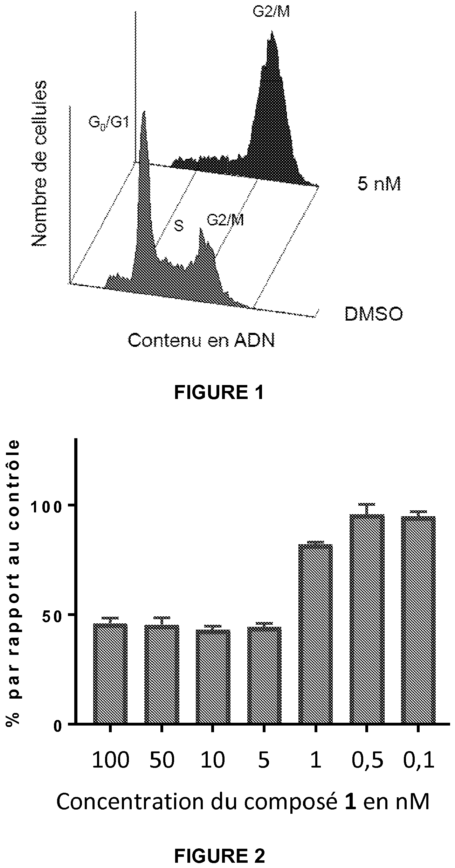

L'analyse par cytométrie en flux des cellules traitées HCT116 avec le composé **1** a montré que ce dernier bloque la division cellulaire en phase G2/M. Cet effet est significatif après 24 heures d'exposition des cellules au composé **1** utilisé à la concentration de 5 nM (Figure 1).

### 2.4. Apoptose

Afin de préciser si le composé **1** entraîne une mort cellulaire par apoptose, l'activité enzymatique intracellulaire des caspases 3 et 7 a été évaluée dans les cultures des cellules HCT116 exposées pendant 24 heures à l'action du composé **1.**

L'apoptose est mesurée en utilisant le kit "Apo-one homogeneous caspase-3/7 assay" (Promega Co, WI, USA) en suivant les recommandations du fabriquant. En bref, les cellules sont ensemencées dans des plaques de culture de 96 puits à raison de 50 000 cellules par puits dans 100 µL de milieu de culture. Après 24 heures d'incubation, le milieu est remplacé par 100 µL de milieu de culture contenant différentes concentrations du composé **1** ou 0,1% de DMSO (contrôle négatif). Après 24 heures de traitement, on ajoute dans chaque puit 100 µL de réactif contenant le substrat des caspases et le tampon de la réaction. Après 1 heure d'incubation, la fluorescence émise par les cellules est mesurée à 527 nm à l'aide d'un lecteur de microplaque de type Victor (Perkin-Elmer, USA).

Les résultats présentés à la Figure 2 montrent que l'incubation des différentes cellules avec le composé **1** conduit à une forte induction de l'apoptose.

### 2.5. Étude in vitro sur la formation de tubes vasculaires sur Matrigel^{®}

Pour savoir si le composé **1** perturbe l'organisation spatiale des cellules endothéliales en structures similaires à des capillaires vasculaires, des cellules endothéliales humaines (HUVECs) ont été traitées immédiatement après la mise en culture sur Matrigel^{™} ou après 24 heures de culture, afin de leur permettre de former des tubes vasculaires.

Les cellules HUVECs (cellules endothéliales humaines issues de cordon ombilical) ont été cultivées dans du milieu de culture EGM2 (Promocell-Allemagne). Les cellules ont été maintenues en culture à 37 °C dans une atmosphère humide contenant 5% de CO₂.

Pour évaluer l'activité anti-vasculaire du composé 1, les cellules HUVECs ont été mise en culture dans des plaques de culture de 96 puits préalablement recouvertes avec un extrait de matrice extracellulaire (Matrigel^{™}, BD Biosciences, Le Pont-de-Claix, France) dans lequel elles forment spontanément des tubes capillaires.

Tout d'abord, nous avons mesuré la capacité du composé **1** à inhiber la formation du réseau capillaire. Le Matrigel^{™} est déposé dans des plaques de culture de 96 puits à raison de 70µL/puits et laissé à incuber à 37°C pendant 45 minutes pour permettre sa polymérisation. 20 000 cellules HUVECs sont ensemencées dans chacun des puits contenant le Matrigel^{™} en absence ou en présence de différentes concentrations du composé **1,** à raison de 3 puits par concentration. Après 2 heures, 3 heures et 5 heures d'incubation à 37°C, les cellules sont observées et photographiées à l'aide d'un microscope optique de type TE2000 (Nikon, France), équipé d'une caméra.

En parallèle, 20 000 cellules HUVECs ont été ensemencées dans chacun des puits contenant le Matrigel^{™}. Après 16 heures d'incubation, quand le réseau capillaire est bien formé, le composé **1** a été ajouté à différentes concentrations. L'effet du produit a été observé après 2 heures, 3 heures et 5 heures d'incubation à l'aide d'un microscope optique.

On peut observer qu'après un traitement de 5 heures à une dose de 10 nM (non toxique), le composé **1** induit une diminution très importante du nombre de tubes vasculaires. Ces résultats indiquent que le composé **1** possède également une activité anti-vasculaire potentiellement utile en thérapeutique.

### 2.6. Test de présentation antigénique in vitro

La lignée MCA205 de fibrosarcome murin a été cultivée à 37 °C sous 5% de CO₂ dans du RPMI-1640 complété avec 10% de sérum de veau fœtal (SVF), 1% L-glutamine, 1% penicillin/streptomycin, 1 mM sodium pyruvate et 1% d'acides aminés non essentiels (MEM non-essential amino acids, Gibco). Les cellules B3Z, hybridomes de cellules T CD8+, ont été cultivées à 37 °C sous 5% de CO₂ dans du RPMI-1640 complété avec 10% de sérum de veau fœtal (SVF), 1% L-glutamine, 1% penicillin/streptomycin et 50 µM β-mercaptoéthanol. La lignée de mélanome murin B16F10 a été cultivée à 37 °C sous 5% de CO₂ dans du DMEM contenant 10% de sérum de veau fœtal (SVF), 1% L-glutamine et 1% penicillin/streptomycin.

Le jour précédent la transfection, les MCA205 ont été transférées en plaque 6 puits à raison de 1,7.10⁵ cellules/puit. Les B16F10 ont été transférées en plaque 6 puits à raison de 1,8.10⁵ cellules/puit. Les MCA205 ont été transfectées avec 1 µg de plasmide (pcDNA3 ou Globine-Intron SL8 représentant la séquence antigénique pouvant être reconnue à la surface cellulaire des cellules l'exprimant par les hybridomes B3Z) en utilisant le réactif jetPRIME selon les consignes données par le fournisseur (Polyplus). Les B16F10 ont été transfectées avec 1 µg de plasmide (pcDNA3 ou Globine-Intron SL8) en utilisant le réactif GeneJuice selon les consignes données par le fournisseur (Novagen).

Les différents composés dilués dans le DMSO ont été ajoutés 24h après la transfection pour 18 h. Par la suite, 50 000 cellules transfectées (MCA205 ou B16F10) ont été mises en culture en présence de 100 000 cellules B3Z dans une plaque 96 puits pendant 18 h. Le peptide SIINFEKL a été ajouté à 1 µg/puits en contrôle. Après 5 minutes de centrifugation à 1200 rpm, les cellules ont été lavées deux fois avec 200 µL de PBS, puis lysées dans 50 µL de tampon de lyse (0,2% TritonX-100, 10 mM DL-Dithiothreitol (Sigma), 90 mM K₂HPO₄ et 8,5 mM KH₂PO₄ dans l'eau). Après 10 min de centrifugation à 3000 rpm, 45 µL de surnageant ont été transférés dans une plaque 96 puits blanche et opaque (OptiPlaque-96, PerkinElmer). 100 µL de tampon de révélation (10 mM MgCl₂, 11,2 mM β-mercaptoéthanol, 0,0015% IGEPAL^{®} CA-630 et 40 µM 4-Methylumbelliferyl β-D-Galactopyranoside (MUG) dans du PBS) ont été ajoutés dans chaque puits pour une incubation d'au moins 3 h à l'abri de la lumière. L'activité β-galactosidase des B3Z est mesurée en fluorescence sur un FLUOstar Optima (BMG Labtech). Le filtre d'excitation est programmé à 355 nm, celui d'émission à 460 nm.

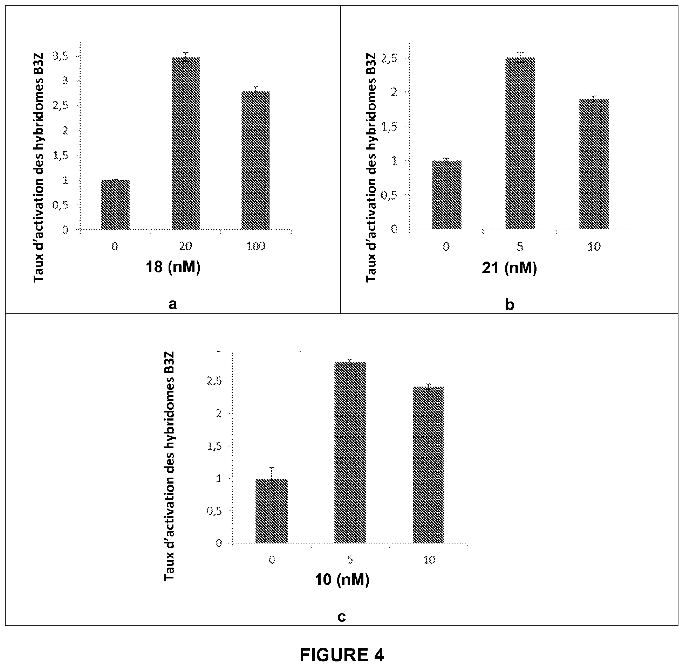

La variation de présentation antigénique dans la lignée du sarcome MCA205 est représentée dans la figure 4 : en fonction de la concentration en composé **18** (figure 4a), **21** (figure 4b) et **10** (figure 4c).

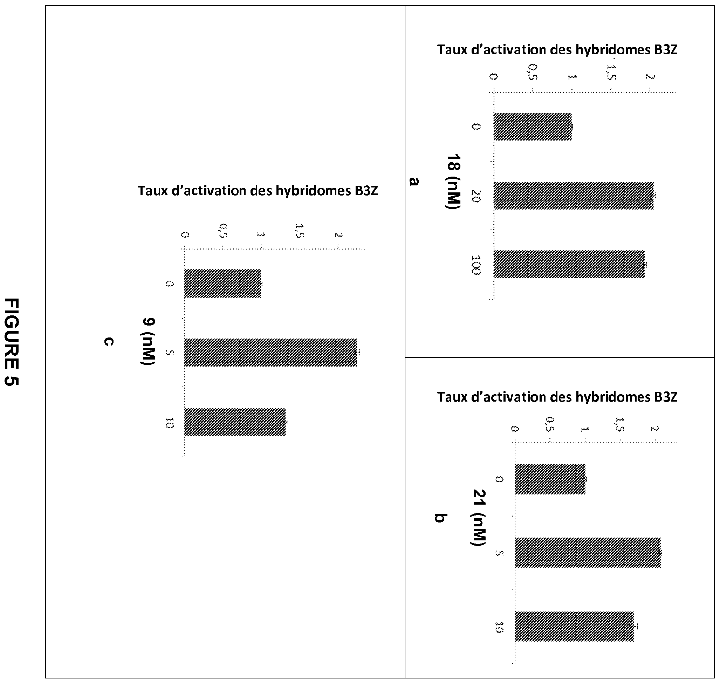

La variation de présentation antigénique dans la lignée du mélanome B16F10 est représentée dans la figure 5 : en fonction de la concentration en composé **18** (figure 5a), **21** (figure 5b) et **9** (figure 5c).

## Revendications

1. Composé de formule (I) suivante : 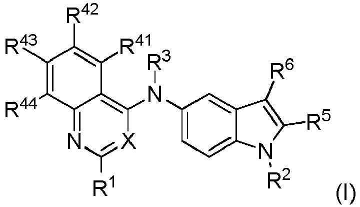 dans laquelle :
- X représente un groupe -CH- ou un atome d'azote,
- R¹ représente un atome d'hydrogène, un atome d'halogène, de préférence un atome de chlore, un groupe (C₁-C₆)alkyle, de préférence un groupe méthyle, un groupe -CN ou un groupe -CF₃,
- R² représente un atome d'hydrogène, un groupe (C₁-C₆)alkyle, un groupe aryle-(Ci-C₆)alkyle, de préférence un benzyle, ou un groupe -COR²¹ avec R²¹ représentant un groupe (C₁-C₆)alkyle ou un groupe aryle,
- R³ représente un groupe (C₁-C₆)alkyle, de préférence un groupe méthyle, un groupe aryle-(C₁-C₆)alkyle ou un groupe -COR³¹ avec R³¹ représentant un groupe (C₁-C₆)alkyle ou un groupe aryle,
- R⁴¹, R⁴², R⁴³ et R⁴⁴ représentent indépendamment les uns des autres un atome d'hydrogène ; un atome d'halogène ; -OR⁴⁵ ; -SR⁴⁵ ; -NR⁴⁵R⁴⁶ ; -NO₂ ; ou un groupe (C₁-C₆)alkyle éventuellement substitué par un ou plusieurs substituants choisis parmi un atome d'halogène, -OR⁴⁵, -SR⁴⁵, -NR⁴⁵R⁴⁶ et -NO₂, avec R⁴⁵ et R⁴⁶ représentant indépendamment l'un de l'autre un atome d'hydrogène, un groupe (C₁-C₆)alkyle, un groupe -CF₃ ou un groupe -COR⁴⁷ avec R⁴⁷ représentant un groupe (C₁-C₆)alkyle ou un groupe aryle,
- R⁵ et R⁶ représentent un atome d'hydrogène ou forment ensemble une chaine -CR⁵¹=CR⁵²-CR⁵³=CR⁵⁴- avec R⁵¹, R⁵², R⁵³ et R⁵⁴ représentant indépendamment les uns des autres un atome d'hydrogène ; un atome d'halogène ; -OR⁵⁵ ; -SR⁵⁵ ; -NR⁵⁵R⁵⁶ ; - NO₂; ou un groupe (C₁-C₆)alkyle éventuellement substitué par un ou plusieurs substituants choisis parmi un atome d'halogène,-OR⁵⁵, -SR⁵⁵, -NR⁵⁵R⁵⁶ et -NO₂, avec R⁵⁵ et R⁵⁶ représentant indépendamment l'un de l'autre un atome d'hydrogène, un groupe (C₁-C₆)alkyle, un groupe -CF₃ ou un groupe -COR⁵⁷ avec R⁵⁷ représentant un groupe (C₁-C₆)alkyle ou un groupe aryle,
ou un sel pharmaceutiquement acceptable de celui-ci,
**caractérisé en ce que**, lorsque X représente un atome d'azote, R¹ représente un atome de chlore, un groupe -CN ou un groupe -CF₃, ou R⁵ et R⁶ forment ensemble une chaine -CR⁵¹=CR⁵²-R⁵³=CR⁵⁴-.

2. Composé selon la revendication 1, **caractérisé en ce que** R¹ représente un atome de chlore, un groupe -CN ou un groupe -CF₃, de préférence R¹ représente un atome de chlore.

3. Composé selon l'une quelconque des revendications 1 et 2, **caractérisé en ce que** R³ représente un groupe (C₁-C₆)alkyle, de préférence un groupe méthyle et/ou R² représente un groupe (C₁-C₆)alkyle, de préférence un groupe méthyle, ou un groupe aryle-(C₁-C₆)alkyle, de préférence un groupe benzyle, de préférence R² représente un groupe (C₁-C₆)alkyle, de préférence un groupe méthyle.

4. Composé selon l'une quelconque des revendications 1 à 3, **caractérisé en ce que** R⁴¹, R⁴², R⁴³ et R⁴⁴ représentent indépendamment les uns des autres un atome d'hydrogène, un atome d'halogène, -OR⁴⁵, -SR⁴⁵, -NR⁴⁵R⁴⁶ ou -NO₂ avec R⁴⁵ et R⁴⁶ représentant indépendamment l'un de l'autre un atome d'hydrogène, un groupe CF₃ ou un groupe (C₁-C₆)alkyle, notamment un atome d'hydrogène ou un groupe (C₁-C₆)alkyle.

5. Composé selon la revendication 4, **caractérisé en ce que** R⁴¹, R⁴², R⁴³ et R⁴⁴ représentent indépendamment les uns des autres un atome d'hydrogène, -OR⁴⁵, -NR⁴⁵R⁴⁶ ou -NO₂ avec R⁴⁵ et R⁴⁶ représentant indépendamment l'un de l'autre un atome d'hydrogène, un groupe (C₁-C₆)alkyle, de préférence, R⁴¹, R⁴², R⁴³ et R⁴⁴ représentent un atome d'hydrogène.

6. Composé selon l'une quelconque des revendications 1 à 5 présentant la formule (la) suivante : 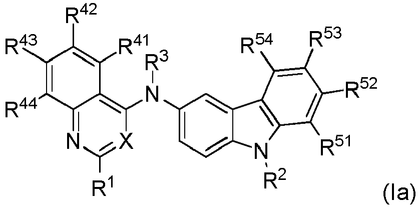 dans laquelle X, R¹, R², R³, R⁴¹, R⁴², R⁴³, R⁴⁴, R⁵¹, R⁵², R⁵³ et R⁵⁴ sont tels que définis à l'une quelconque des revendications 1 à 5,
ou un sel pharmaceutiquement acceptable de celui-ci.

7. Composé selon l'une quelconque des revendications 1 à 6, **caractérisé en ce que** R⁵¹, R⁵², R⁵³ et R⁵⁴ représentent indépendamment les uns des autres un atome d'hydrogène, -OR⁵⁵, -NR⁵⁵R⁵⁶, -NO₂ ou un groupe (C₁-C₆)alkyle éventuellement substitué par un ou plusieurs substituants choisis parmi un atome d'halogène, un groupe -OR⁵⁵, -SR⁵⁵, -NR⁵⁵R⁵⁶ et -NO₂, avec R⁵⁵ et R⁵⁶ représentant indépendamment l'un de l'autre un atome d'hydrogène ou un groupe (C₁-C₆)alkyle.

8. Composé selon l'une quelconque des revendications 1 à 6, **caractérisé en ce que** R⁵¹, R⁵², R⁵³ et R⁵⁴ représentent indépendamment les uns des autres un atome d'hydrogène, un atome d'halogène, -OR⁵⁵, -NR⁵⁵R⁵⁶, -NO₂ ou un groupe (C₁-C₆)alkyle éventuellement substitué par un groupe -OR⁵⁵, avec R⁵⁵ et R⁵⁶ représentant indépendamment l'un de l'autre un atome d'hydrogène un groupe CF₃ ou un groupe (C₁-C₆)alkyle, notamment un atome d'hydrogène ou un groupe (C₁-C₆)alkyle.

9. Composé selon l'une quelconque des revendications 1 à 5, **caractérisé en ce que** R⁵ et R⁶ représentent un atome d'hydrogène.

10. Composé selon la revendication 1 présentant la formule (Ib) suivante :

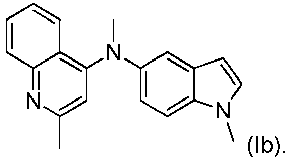

11. Composé selon la revendication 1 choisi parmi : 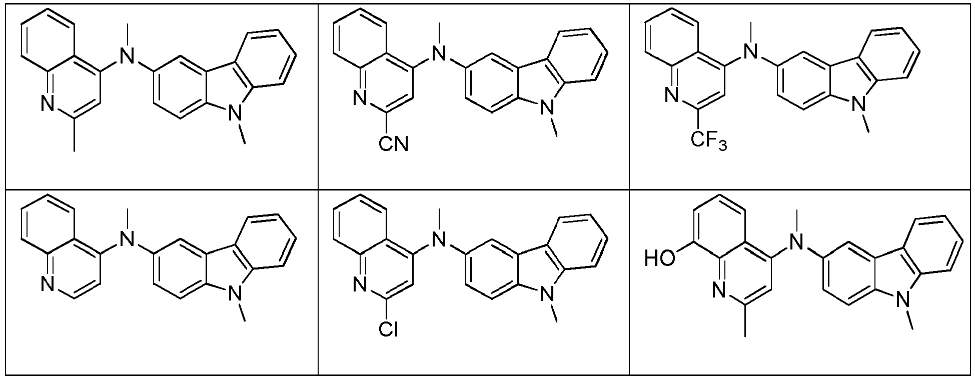 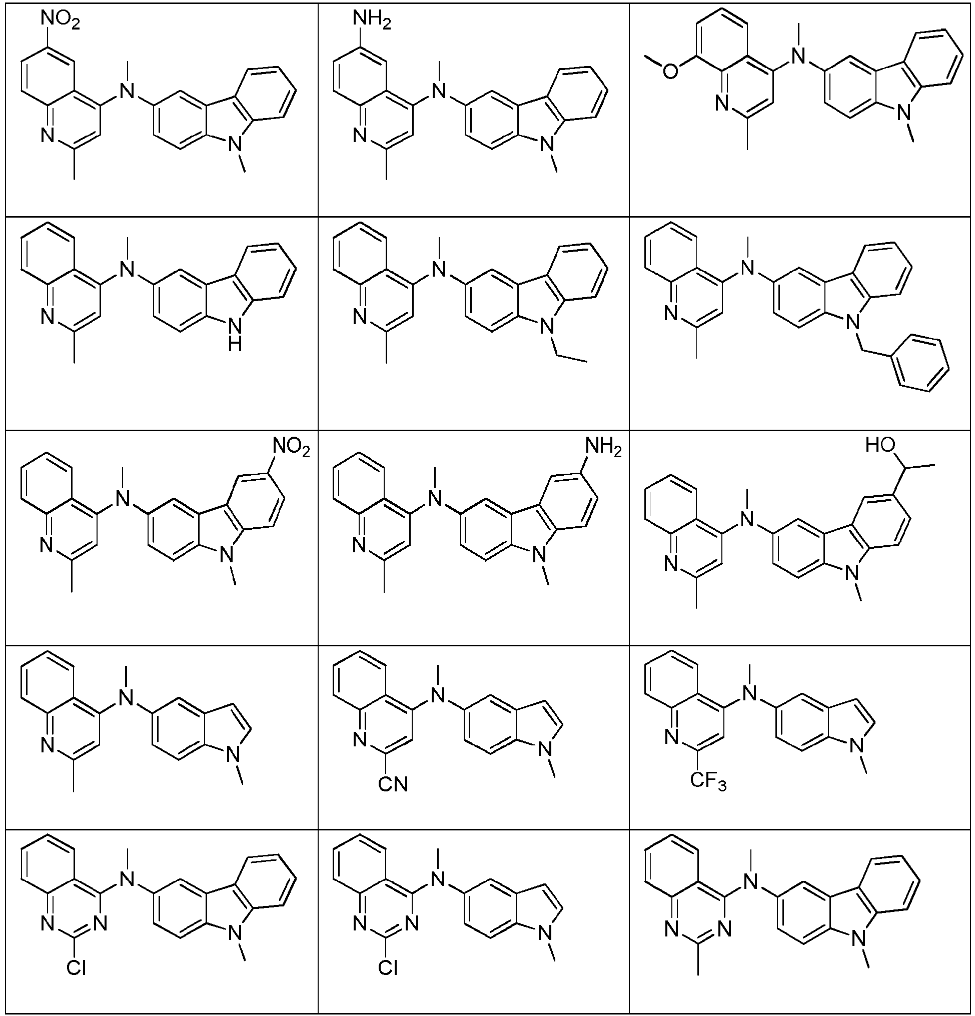 et leurs sels pharmaceutiquement acceptables.

12. Composé selon l'une quelconque des revendications 1 à 11 pour son utilisation en tant que médicament.

13. Composé selon l'une quelconque des revendications 1 à 11 pour son utilisation en tant que médicament destiné à traiter le cancer.

14. Composition pharmaceutique comprenant au moins un composé selon l'une quelconque des revendications 1 à 11 et un excipient pharmaceutiquement acceptable.

15. Procédé de préparation d'un composé selon l'une quelconque des revendications 1 à 11 ou d'un sel pharmaceutiquement acceptable de celui-ci comprenant les étapes suivantes :
(1) couplage d'un dérivé aminé de formule (II) suivante : 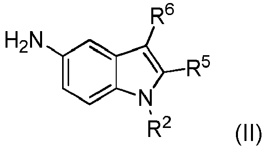 dans laquelle R², R⁵ et R⁶ sont tels que définis à la revendication 1,
avec un composé de formule (III) suivante : 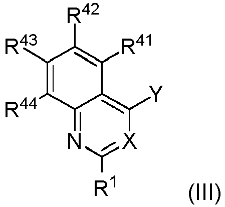 dans laquelle Y représente un atome de chlore, de brome, d'iode, un groupe trifluorométhylsulfonate ou un groupe tosyle et X, R¹, R⁴¹, R⁴², R⁴³ et R⁴⁴ sont tels que définis à la revendication 1,
pour donner un composé de formule (I) selon la revendication 1 avec R³ = H,
(2) substitution du groupe R³ = H porté par l'amine du composé de formule (I) obtenu à l'étape (1) qui précède pour donner un composé de formule (I) selon la revendication 1 avec R³ représentant un groupe (C₁-C₆)alkyle, un groupe aryle-(C₁-C₆)alkyle ou un groupe -COR³¹ avec R³¹ tel que défini dans la revendication 1, et
(3) éventuellement salification du composé de formule (I) obtenu à l'étape (1) ou (2) qui précède pour donner un sel pharmaceutiquement acceptable d'un composé de formule (I) selon la revendication 1.

## Patentansprüche

1. Verbindung der folgenden Formel (I): 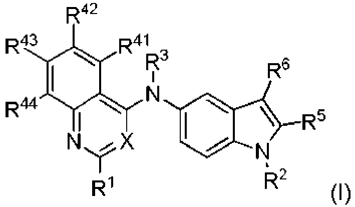 wobei:
- X eine -CH- Gruppe oder ein Stickstoffatom darstellt,
- R¹ ein Wasserstoffatom, ein Halogenatom, vorzugsweise ein Chloratom, eine (C₁-C₆)Alkylgruppe, vorzugsweise eine Methylgruppe, eine -CN Gruppe oder eine -CF₃ Gruppe darstellt,
- R² ein Wasserstoffatom, eine (C₁-C₆)Alkylgruppe, eine Aryl- (C₁-C₆)Alkylgruppe, vorzugsweise ein Benzyl, oder eine -COR²¹ Gruppe und R²¹ eine (C₁-C₆) Alkylgruppe oder eine Arylgruppe darstellt,
- R³ eine (C₁-C₆)Alkylgruppe, vorzugsweise eine Methylgruppe, eine Aryl- (C₁-C₆)Alkylgruppe oder eine -COR³¹ Gruppe darstellt, wobei R³¹ eine (C₁-C₆)Alkylgruppe oder eine Arylgruppe darstellt,
- R⁴¹, R⁴², R⁴³ und R⁴⁴ unabhängig voneinander ein Wasserstoffatom; ein Halogenatom; -OR⁴⁵; -SR⁴⁵; -NR⁴⁵R⁴⁶; -NO₂; oder eine (C₁-C₆)Alkylgruppe, gegebenenfalls substituiert durch einen oder mehrere aus einem Halogenatom, -OR⁴⁵, -SR⁴⁵, - NR⁴⁵R⁴⁶ und -NO₂ ausgewählte Substituenten, sowie R⁴⁵ und R⁴⁶ unabhängig voneinander ein Wasserstoffatom, eine (C₁-C₆)Alkylgruppe, eine -CF₃ Gruppe oder eine -COR⁴⁷ Gruppe und R⁴⁷ eine (C₁-C₆)Alkylgruppe oder eine Arylgruppe darstellen,
- R⁵ und R⁶ ein Wasserstoffatom darstellen oder zusammen eine -CR⁵¹=CR⁵²-CR⁵³=CR⁵⁴- Kette bilden, wobei R⁵¹, R⁵², R⁵³ und R⁵⁴, unabhängig voneinander ein Wasserstoffatom; ein Halogenatom; -OR⁵⁵; -SR⁵⁵; -NR⁵⁵R⁵⁶; -NO₂; oder eine (C₁-C₆)Alkylgruppe, gegebenenfalls substituiert durch einen oder mehrere aus einem Halogenatom, -OR⁵⁵, - SR⁵⁵, -NR⁵⁵R⁵⁶ und -NO₂ ausgewählte Substituenten, sowie R⁵⁵ und R⁵⁶, unabhängig voneinander ein Wasserstoffatom, eine (C₁-C₆)Alkylgruppe, eine -CF₃ Gruppe oder eine -COR⁵⁷ Gruppe und R⁵⁷ eine (C₁-C₆)Alkylgruppe oder eine Arylgruppe darstellen,
oder ein pharmazeutisch verträgliches Salz davon,
**dadurch gekennzeichnet, dass**, wenn X ein Stickstoffatom, R¹ ein Chloratom, eine -CN Gruppe oder eine -CF₃ Gruppe darstellt bzw. R⁵ und R⁶ zusammen eine -CR⁵¹=CR⁵²-CR⁵³=CR⁵⁴- Kette bilden.

2. Verbindung nach Anspruch 1, **dadurch gekennzeichnet, dass** R¹ ein Chloratom, eine -CN Gruppe oder eine -CF₃ Gruppe darstellt, vorzugsweise R¹ = Chloratom.

3. Verbindung nach einem der Ansprüche 1 und 2, **dadurch gekennzeichnet, dass** R³ eine (C₁-C₆)Alkylgruppe, vorzugsweise eine Methylgruppe, und/oder R² eine (C₁-C₆)Alkylgruppe, vorzugsweise eine Methylgruppe, oder eine Aryl-(C₁-C₆)Alkylgruppe, vorzugsweise eine Benzylgruppe, R² vorzugsweise eine (C₁-C₆)Alkylgruppe, vorzugsweise eine Methylgruppe, darstellt.

4. Verbindung nach einem der Ansprüche 1 bis 3, **dadurch gekennzeichnet, dass** R⁴¹, R⁴², R⁴³ und R⁴⁴ unabhängig voneinander ein Wasserstoffatom, ein Halogenatom, -OR⁴⁵, -SR⁴⁵, - NR⁴⁵R⁴⁶ oder -NO₂ darstellen, wobei R⁴⁵ und R⁴⁶ unabhängig voneinander ein Wasserstoffatom, eine CF₃ Gruppe oder eine (C₁-C₆) Alkylgruppe, insbesondere ein Wasserstoffatom oder eine (C₁-C₆)Alkylgruppe, darstellen.

5. Verbindung nach Anspruch 4, **dadurch gekennzeichnet, dass** R⁴¹, R⁴², R⁴³ und R⁴⁴ unabhängig voneinander ein Wasserstoffatom, -OR⁴⁵, -NR⁴⁵R⁴⁶ oder -NO₂ darstellen, wobei R⁴⁵ und R⁴⁶ unabhängig voneinander ein Wasserstoffatom oder eine (C₁-C₆)Alkylgruppe und vorzugsweise R⁴¹, R⁴², R⁴³ und R⁴⁴ ein Wasserstoffatom darstellen.

6. Verbindung nach einem der Ansprüche 1 bis 5 mit folgender Formel (Ia): 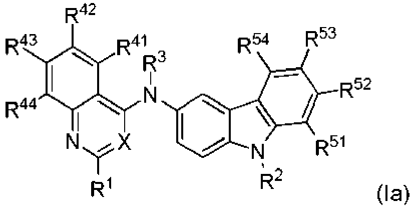 wobei X, R¹, R², R³, R⁴¹, R⁴², R⁴³, R⁴⁴, R⁵¹, R⁵², R⁵³ und R⁵⁴ nach einem der Ansprüche 1 bis 5 definiert sind, oder ein pharmazeutisch verträgliches Salz davon.

7. Verbindung nach einem der Ansprüche 1 bis 6, **dadurch gekennzeichnet, dass** R⁵¹, R⁵², R⁵³ und R⁵⁴ unabhängig voneinander ein Wasserstoffatom, -OR⁵⁵, -NR⁵⁵R⁵⁶, -NO₂ oder eine (C₁-C₆)Alkylgruppe, gegebenenfalls substituiert durch einen oder mehrere aus einem Halogenatom, - OR⁵⁵, -SR⁵⁵, -NR⁵⁵R⁵⁶ -NO₂ ausgewählte Substituenten, darstellen, wobei R⁵⁵ und R⁵⁶ unabhängig voneinander ein Wasserstoffatom oder eine (C₁-C₆)Alkylgruppe darstellen.

8. Verbindung nach einem der Ansprüche 1 bis 6, **dadurch gekennzeichnet, dass** R⁵¹, R⁵², R⁵³ und R⁵⁴ unabhängig voneinander ein Wasserstoffatom, ein Halogenatom, -OR⁵⁵, -NR⁵⁵R⁵⁶, - NO₂ oder eine (C₁-C₆) Alkylgruppe, gegebenenfalls durch eine -OR⁵⁵ Gruppe substituiert, darstellen, wobei R⁵⁵ und R⁵⁶ unabhängig voneinander ein Wasserstoffatom, eine CF₃ Gruppe oder eine (C₁-C₆)Alkylgruppe, insbesondere ein Wasserstoffatom oder eine (C₁-C₆)Alkylgruppe, darstellen.

9. Verbindung nach einem der Ansprüche 1 bis 5, **dadurch gekennzeichnet, dass** R⁵ und R⁶ ein Wasserstoffatom darstellen.

10. Verbindung nach Anspruch 1 mit folgender Formel (Ib):

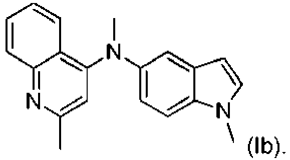

11. Verbindung nach Anspruch 1 ausgewählt aus: 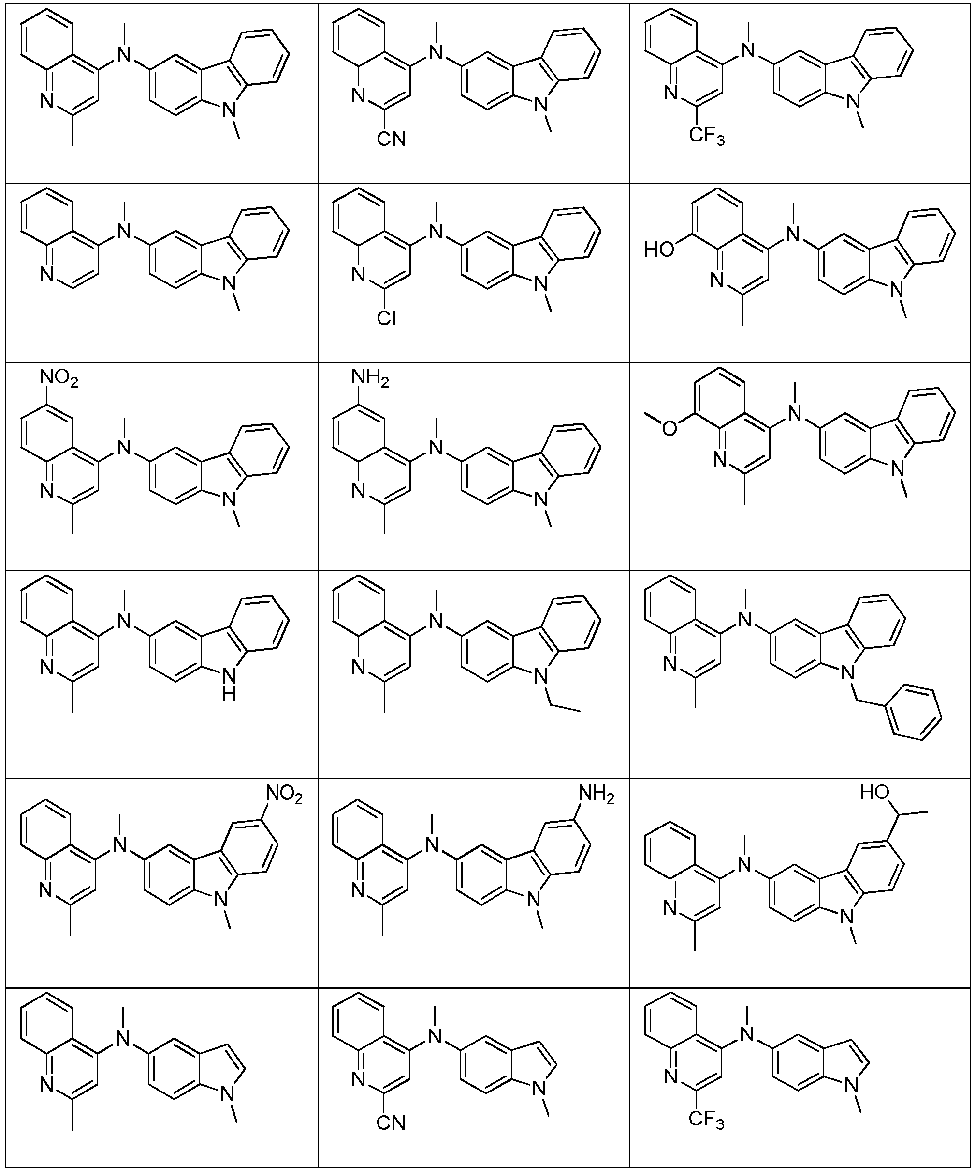 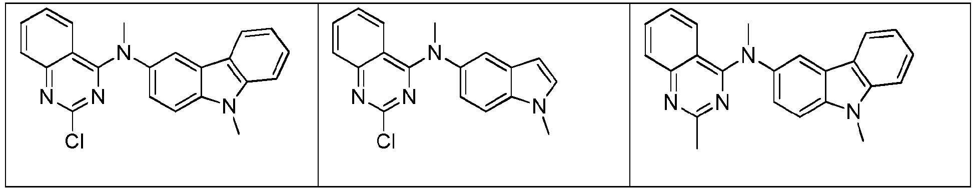 und ihren pharmazeutisch verträglichen Salzen.

12. Verbindung nach einem der Ansprüche 1 bis 11 zur Verwendung als Arzneimittel.

13. Verbindung nach einem der Ansprüche 1 bis 11 zur Verwendung als Krebsmedikament.

14. Pharmazeutische Zusammensetzung, die mindestens eine Verbindung nach einem der Ansprüche 1 bis 11 und einen pharmazeutisch verträglichen Trägerstoff beinhaltet.

15. Verfahren zur Herstellung einer Verbindung nach einem der Ansprüche 1 bis 11 oder eines pharmazeutisch verträglichen Salzes davon, das die folgenden Schritte beinhaltet:
(1) Kopplung eines Aminoderivats mit folgender Formel (II): 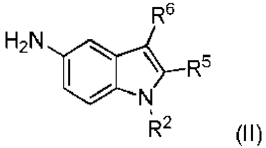 wobei R², R⁵ und R⁶ der Definition in Anspruch 1 entsprechen,
mit einer Verbindung der folgenden Formel (III) : 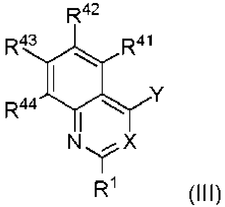 wobei Y ein Chlor-, Brom- oder Jodatom, eine Trifluormethylsulfonat- oder Tosylgruppe darstellt und X-, R¹, R⁴¹, R⁴², R⁴³ und R⁴⁴ der Definition in Anspruch 1 entsprechen,
um eine Verbindung der Formel (I) nach Anspruch 1 zu erhalten, wobei R³ = H,
(2) Substitution der Gruppe R³ = H, getragen durch das Amin der Verbindung der Formel (I), die in Schritt (1) oben gewonnen wurde, um eine Verbindung der Formel (I) nach Anspruch 1 zu erhalten, wobei R³ eine (C₁-C₆)Alkylgruppe, eine Aryl-(C₁-C₆)Alkylgruppe oder eine -COR³¹ Gruppe darstellt, wobei R³¹ der Definition in Anspruch 1 entspricht, und
(3) gegebenenfalls Versalzung der Verbindung der Formel (I), die in Schritt (1) oder (2) gewonnen wurde, um ein pharmazeutisch verträgliches Salz aus einer Verbindung der Formel (I) nach Anspruch 1 zu erhalten.

## Claims

1. A compound of following formula (I): 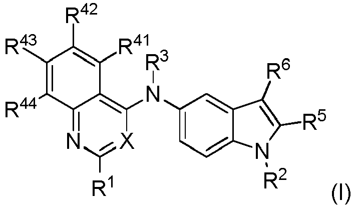 in which:
- X represents a -CH- group or a nitrogen atom,
- R¹ represents a hydrogen atom, a halogen atom, preferably a chlorine atom, a (C₁-C₆)alkyl group, preferably a methyl group, a -CN group or a -CF₃ group,
- R² represents a hydrogen atom, a (C₁-C₆)alkyl group, an aryl-(C₁-C₆)alkyl group, preferably a benzyl, or a -COR²¹ group with R²¹ representing a (C₁-C₆)alkyl group or an aryl group,
- R³ represents a (C₁-C₆)alkyl group, preferably a methyl group, an aryl-(C₁-C₆)alkyl group or a -COR³¹ group with R³¹ representing a (C₁-C₆)alkyl group or an aryl group,
- R⁴¹, R⁴², R⁴³ and R⁴⁴ represent independently of each other a hydrogen atom; a halogen atom; -OR⁴⁵ ; -SR⁴⁵ ; -NR⁴⁵R⁴⁶; -NO₂; or a (C₁-C₆)alkyl group optionally substituted by one or more substituents selected from among a halogen atom, - OR⁴⁵, -SR⁴⁵, -NR⁴⁵R⁴⁶ and -NO₂, with R⁴⁵ and R⁴⁶ representing independently of each other a hydrogen atom, a (C₁-C₆)alkyl group, a -CF₃ group or a -COR⁴⁷ group with R⁴⁷ representing a (C₁-C₆)alkyl group or an aryl group,
- R⁵ and R⁶ represent a hydrogen atom or form together a -CR⁵¹=CR⁵²-CR⁵³=CR⁵⁴- chain with R⁵¹, R⁵², R⁵³ and R⁵⁴ representing independently of each other a hydrogen atom; a halogen atom; -OR⁵⁵ ; -SR⁵⁵ ; -NR⁵⁵R⁵⁶ ; -NO₂; or a (C₁-C₆)alkyl group optionally substituted by one or more substituents selected from among a halogen atom,-OR⁵⁵, -SR⁵⁵, -NR⁵⁵R⁵⁶ and -NO₂, with R⁵⁵ and R⁵⁶ representing independently of each other a hydrogen atom, a (C₁-C₆)alkyl group, a -CF₃ group or a -COR⁵⁷ group with R⁵⁷ representing a (C₁-C₆)alkyl group or an aryl group,
or a pharmaceutically acceptable salt thereof,
**characterised in that**, when X represents a nitrogen atom, R¹ represents a chlorine atom, a -CN group or a -CF₃ group, or R⁵ and R⁶ form together a -CR⁵¹=CR⁵²-CR⁵³=CR⁵⁴- chain.

2. The compound according to claim 1, **characterised in that** R¹ represents a chlorine atom, a -CN group or a -CF₃ group, preferably R¹ represents a chlorine atom.

3. The compound according to any one of claims 1 and 2, **characterised in that** R³ represents a (C₁-C₆)alkyl group, preferably a methyl group and/or R² represents a (C₁-C₆)alkyl group, preferably a methyl group, or an aryl-(C₁-C₆)alkyl group, preferably a benzyl group, preferably R² represents a (C₁-C₆)alkyl group, preferably a methyl group.

4. The compound according to any one of claims 1 to 3, **characterised in that** R⁴¹, R⁴², R⁴³ and R⁴⁴ represent independently of each other a hydrogen atom, a halogen atom, -OR⁴⁵, - SR⁴⁵, -NR⁴⁵R⁴⁶ or -NO₂ with R⁴⁵ and R⁴⁶ representing independently of each other a hydrogen atom, a CF₃ group or a (C₁-C₆)alkyl group, notably a hydrogen atom or a (C₁-C₆)alkyl group.

5. The compound according to claim 4, **characterised in that** R⁴¹, R⁴², R⁴³ and R⁴⁴ represent independently of each other a hydrogen atom, -OR⁴⁵, -NR⁴⁵R⁴⁶ or -NO₂ with R⁴⁵ and R⁴⁶ representing independently of each other a hydrogen atom, a (C₁-C₆)alkyl group, preferably, R⁴¹, R⁴², R⁴³ and R⁴⁴ represent a hydrogen atom.

6. The compound according to any one of claims 1 to 5 having following formula (Ia): 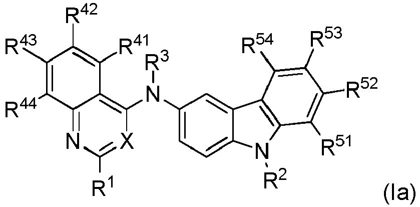 in which X, R¹, R², R³, R⁴¹, R⁴², R⁴³, R⁴⁴, R⁵¹, R⁵², R⁵³ and R⁵⁴ are as defined in any one of claims 1 to 5,
or a pharmaceutically acceptable salt thereof.

7. The compound according to any one of claims 1 to 6, **characterised in that** R⁵¹, R⁵², R⁵³ and R⁵⁴ represent independently of each other a hydrogen atom, -OR⁵⁵, -NR⁵⁵R⁵⁶, -NO₂ or a (C₁-C₆)alkyl group optionally substituted by one or more substituents selected from among a halogen atom, a -OR⁵⁵, -SR⁵⁵, -NR⁵⁵R⁵⁶ and -NO₂ group, with R⁵⁵ and R⁵⁶ representing independently of each other a hydrogen atom or a (C₁-C₆)alkyl group.

8. The compound according to any one of claims 1 to 6, **characterised in that** R⁵¹, R⁵², R⁵³ and R⁵⁴ represent independently of each other a hydrogen atom, a halogen atom, -OR⁵⁵, - NR⁵⁵R⁵⁶, -NO₂ or a (C₁-C₆)alkyl group optionally substituted by a -OR⁵⁵ group, with R⁵⁵ and R⁵⁶ representing independently of each other a hydrogen atom, a CF₃ group or a (C₁-C₆)alkyl group, notably a hydrogen atom or a (C₁-C₆)alkyl group.

9. The compound according to any one of claims 1 to 5, **characterised in that** R⁵ and R⁶ represent a hydrogen atom.

10. The compound according to claim 1 having the following formula (Ib):

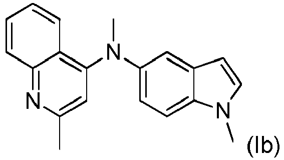

11. The compound according to claim 1 selected from among: 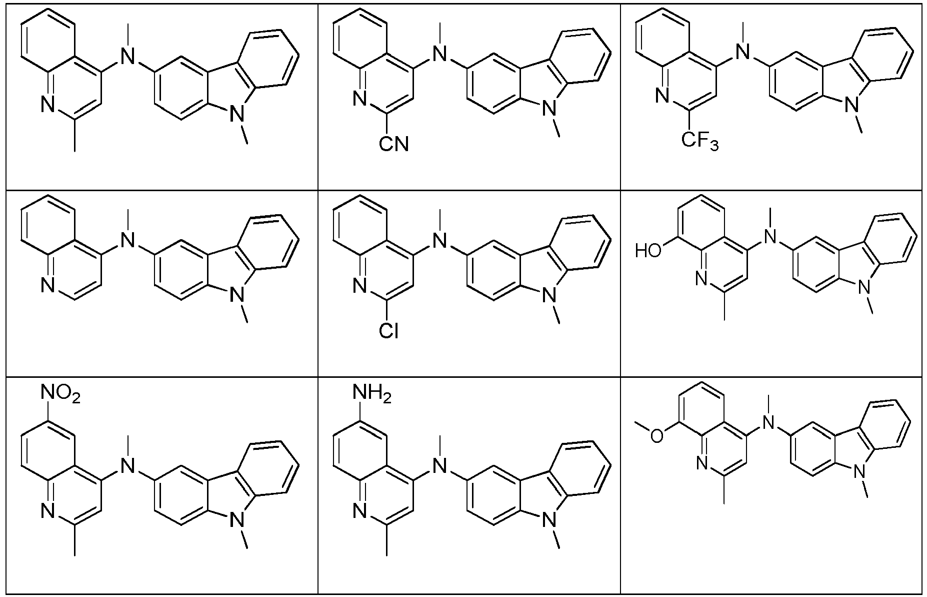 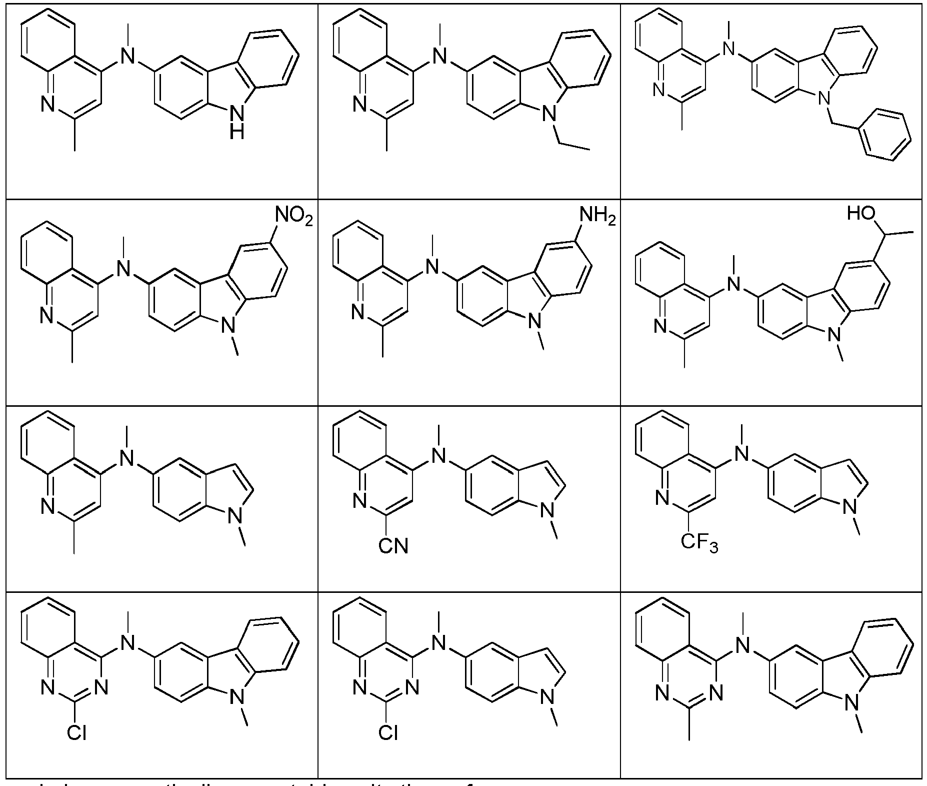 and pharmaceutically acceptable salts thereof.

12. The compound according to any one of claims 1 to 11 for use as a drug.

13. The compound according to any one of claims 1 to 11 for use as a drug intended to treat cancer.

14. A pharmaceutical composition comprising at least one compound according to any one of claims 1 to 11 and a pharmaceutically acceptable excipient.

15. A method for preparing a compound according to any one of claims 1 to 11 or a pharmaceutically acceptable salt thereof comprising the following steps:
(1) coupling an amine derivative of following formula (II): 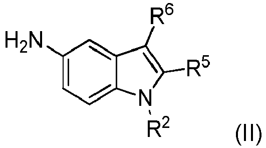 in which R², R⁵ and R⁶ are as defined in claim 1,
with a compound of following formula (III): 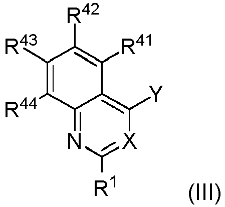 in which Y represents an atom of chlorine, bromine, iodine, a trifluoromethylsulphonate group or a tosyl group and X, R¹, R⁴¹, R⁴², R⁴³ and R⁴⁴ are as defined in claim 1,
to give a compound of formula (I) according to claim 1 with R³ = H,
(2) substituting the R³ = H group borne by the amine of the compound of formula (I) obtained at step (1) which precedes to give a compound of formula (I) according to claim 1 with R³ representing a (C₁-C₆)alkyl group, an aryl-(C₁-C₆)alkyl group or a -COR³¹ group with R³¹ as defined in claim 1, and
(3) optionally salifying the compound of formula (I) obtained at step (1) or (2) which precedes to give a pharmaceutically acceptable salt of a compound of formula (I) according to claim 1.
